(19) Europäisches Patentamt
European Patent Office
Office européen des brevets

(11) Veröffentlichungsnummer: **0 038 458**
**B1**

# (12) EUROPÄISCHE PATENTSCHRIFT

(45) Veröffentlichungstag der Patentschrift: **21.09.83**

(51) Int. Cl.³: **C 07 D 275/06,** A 23 L 1/236

(21) Anmeldenummer: **81102567.5**

(22) Anmeldetag: **06.04.81**

## (54) 4-Hydroxy-1,2-benzisothiazol-3(2H)-on-1,1-dioxide, ihre Salze, ihre Herstellung und Verwendung.

(30) Priorität: **19.04.80 DE 3015113**

(43) Veröffentlichungstag der Anmeldung: **28.10.81 Patentblatt 81/43**

(45) Bekanntmachung des Hinweises auf die Patenterteilung: **21.09.83 Patentblatt 83/38**

(84) Benannte Vertragsstaaten: **AT BE CH DE FR GB IT LI LU NL SE**

(56) Entgegenhaltungen:
**CHEMICAL ABSTRACTS, Band 32, Nr. 10, 20-05-1938, Seiten 3761–3762 Zusammenfassung 37621-7 Columbus, Ohio, US C. FINZI et al.: «Chemical constitution and sweet taste»**

(73) Patentinhaber: **Dr. Karl Thomae GmbH, Postfach 1755, D-7950 Biberach (Riss) (DE)**

(72) Erfinder: **Trummlitz, Günter, Dr. Dipl.-Chem., Buchenweg 27, D-7951 Warthausen (DE)**
Erfinder: **Eberlein, Wolfgang, Dr. Dipl.-Chem., Obere Au 6, D-7950 Biberach 1 (DE)**
Erfinder: **Engel, Wolfgang, Dr. Dipl.-Chem., Mozartstrasse 13, D-7950 Biberach 1 (DE)**
Erfinder: **Schmidt, Günther, Dr. Dipl.-Chem., Johann-Sebastian-Bach-Strasse 27, D-7950 Biberach 1 (DE)**

**EP 0 038 458 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 4-Hydroxy-1,2-benzisothiazol-3(2H)-on-1,1-dioxide, ihre Salze, ihre Herstellung und Verwendung

Die vorliegende Erfindung betrifft als neue Verbindungen 4-Hydroxy-1,2-benzisothiazol-3(2H)-on-1,1-dioxide der allgemeinen Formel I

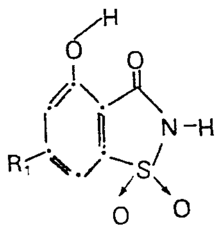

(I)

in der $R_1$ ein Wasserstoffatom oder die Hydroxygruppe bedeutet, ihre physiologisch unbedenklichen Alkali- oder Erdalkalisalze, Verfahren zur Herstellung dieser Verbindungen, diese Verbindungen enthaltende Zubereitungsformen und die Verwendung als Süssstoffe.

Als Salze der 4-Hydroxy-1,2-benzisothiazol-3(2H)-on-1,1-dioxide kommen die physiologisch unbedenklichen Alkali- oder Erdalkalisalze in Frage. Es handelt sich hierbei um die Alkalisalze, wie das Natrium-, Kalium- oder Ammoniumsalz, insbesondere aber das Natriumsalz und um die Erdalkalisalze, wie das Calciumsalz.

An Süssstoffe sind im allgemeinen folgende Anforderungen zu stellen:

a) der Süssstoff muss, als Zusatz zu Nahrungsmitteln, absolut sicher sein, er darf keinerlei toxische oder pharmakologische Wirkungen zeigen;

b) der Süssstoff muss einwandfreie Geschmacksqualitäten aufweisen, wobei die diesbezüglichen Eigenschaften der Saccharose als Vorbild gelten; der Süssstoff darf also keinen Beigeschmack oder Nachgeschmack erzeugen, sein süsser Geschmack muss sofort einsetzen und rasch wieder verschwinden;

c) der Süssstoff muss für seine Anwendung genügend in Wasser löslich, in heissen Getränken temperaturstabil und in sauren Fruchtsaftgetränken säurestabil sein.

Keiner der heute bekannten Süssstoffe erfüllt diese obengenannten Anforderungen vollständig; so ist die Verwendung von Cyclamat und Saccharin als Süssstoff nicht unbedenklich, wie einige toxikologische Befunde bei höheren Dosierungen dieser Süssstoffe zeigten. Süssstoffe mit Dipeptidstruktur sind nicht genügend temperatur- und säurestabil, während Oxathiazinonderivate geschmacklich nicht voll befriedigen. Höhermolekularen Naturstoffen mangelt es an genügender Stabilität, sie sind zudem aufgrund ihres lang andauernden Nachgeschmacks nur bedingt verwendbar.

Es wurde nun gefunden, dass die 4-Hydroxy-1,2-benzisothiazol-3(2H)-on-1,1-dioxide der allgemeinen Formel I und ihre physiologisch unbedenklichen Alkali- oder Erdalkalisalze den bisher verwendeten Süssstoffen bezüglich der Süsskraft mindestens gleichwertig sind, eine einwandfreie Geschmacksqualität ohne Bei- oder Nachgeschmack besitzen, in Wasser sehr gut löslich und darüberhinaus temperatur- und säurestabil sind. Des weiteren fehlt diesen Verbindungen jegliche toxische und pharmakologische Wirkung, so dass sie absolut sicher in ihrer Handhabung sind.

Die erfindungsgemässen Verbindungen der allgemeinen Formel I lassen sich wie folgt herstellen:

1) Durch alkalische Verseifung von 3-Amino-1,2-benzisothiazol-1,1-dioxiden der allgemeinen Formel II

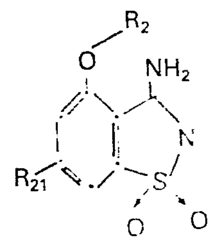

(II)

in der $R_2$ ein Wasserstoffatom, eine Alkyl- oder Aralkylgruppe, vorzugsweise eine Alkylgruppe mit 1 bis 6 C-Atomen oder die Benzyl- oder Phenyläthylgruppe, und $R_{21}$ ein Wasserstoffatom, eine Hydroxy-, eine Alkoxy- oder Aralkoxygruppe, wobei die Alkoxygruppe vorzugsweise 1 bis 6 C-Atome enthält und die Aralkoxygruppe vorzugsweise die Benzyloxy- oder Phenyläthyloxygruppe darstellt, bedeuten.

Ist $R_2$ ein Wasserstoffatom, und $R_{21}$ ein Wasserstoffatom oder eine Hydroxygruppe, so entsteht bei der Verseifung dieser 3-Amino-Verbindung sofort das Endprodukt der allgemeinen Formel I bzw. ihr Salz; ist dagegen $R_2$ eine Alkyl- oder Aralkylgruppe und/oder $R_{21}$ eine Alkoxy- oder Aralkoxygruppe so werden anschliessend diese Alkyl- oder Aralkylgruppen durch eine Etherspaltung abgetrennt.

Die Verseifung einer Verbindung der allgemeinen Formel II wird mit Basen durchgeführt. So behandelt man eine wässrige Lösung einer Verbindung der allgemeinen Formel II mit einer anorganischen Base, beispielsweise mit einem Alkalihydroxid, wie Natrium- oder Kaliumhydroxid, bei Temperaturen zwischen 50 °C und dem Siedepunkt der Lösung. Ist $R_2$ in der Verbindung der allgemeinen Formel II ein Wasserstoffatom oder $R_{21}$ eine Hydroxygruppe, so sind mindestens zwei Äquivalente an Base notwendig, bedeutet aber $R_2$ ein Wasserstoffatom und $R_{21}$ eine Hydroxygruppe so braucht man mindestens drei Äquivalente an Base, ansonsten kommt man mit einem Äquivalent oder einem kleinen Überschuss Base aus. Nach der Verseifung wird angesäuert, und, falls das Produkt nicht von selbst ausfällt, das wässrige Medium mit einem Extraktionsmittel, wie Ethylacetat, ausgeschüttelt.

Die anschliessende Etherspaltung bei einer so erhaltenen Verbindung, die in 4- und/oder in 6-Stellung noch einen Alkoxy- oder Aralkoxyrest enthält, erfolgt mit sauren oder alkalischen Methoden. Die saure Methode umfasst die Anwendung von Lewis-Säuren, wie Borhalogenide, Aluminiumhalogenide oder Pyridinhydrochlorid. Verwendet man Borhalogenide, beispielsweise Bortribromid oder Bortrichlorid, so arbeitet man in aprotischen Lösungsmitteln, z.B. in Halogenkohlenwasserstoffen, bei Temperaturen bis zum Siedepunkt des Reaktionsgemisches. Das Borhalogenid wird vorteilhafterweise in einem molaren Überschuss eingesetzt. Arbeitet man mit Aluminiumhalogeniden, z.B. mit wasserfreiem Aluminiumchlorid, so dienen als aprotische Lösungsmittel vorteilhafterweise Schwefelkohlenstoff, Nitrobenzol oder Toluol. Die Etherspaltung mit Pyridin-hydrochlorid wird vorzugsweise ohne Lösungsmittel in der Schmelze durchgeführt.

Die alkalische Methode bedient sich polarer aprotischer Lösungsmittel, besonders geeignet ist hierfür Dimethylsulfoxid, wobei diese Methode die Anwendung von Halogeniden bzw. Pseudohalogeniden umfasst, die in molaren Mengen oder in einem 5- bis 10-fachen molaren Überschuss eingesetzt werden. Die erforderlichen Reaktionstemperaturen liegen in einem Bereich zwischen 120 und 200 °C, vorzugsweise aber zwischen 150 bis 180 °C. Als Halogenide dienen vorzugsweise Alkalihalogenide, wie Lithiumjodid, -bromid, -chlorid, Natriumchlorid, -bromid, -jodid, Kaliumchlorid, -bromid, -jodid, insbesondere aber Kaliumbromid, als Alkali-pseudohalogenide, die entsprechenden Cyanide oder Rhodanide, insbesondere aber Natrium- und Kaliumcyanid oder Kaliumrhodanid;

2) Durch Oxidation von o-Sulfamoyl-toluolen der allgemeinen Formel III

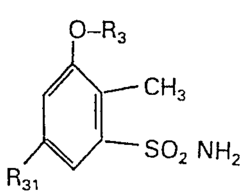

(III)

in der $R_3$ eine Alkyl- oder Aralkylgruppe, vorzugsweise eine Alkylgruppe mit 1 bis 6 Kohlenstoffatomen oder die Benzyl- oder Phenyläthylgruppe und $R_{31}$ ein Wasserstoffatom oder eine Alkoxy- oder Aralkoxygruppe, wobei die Alkoxygruppe vorzugsweise 1 bis 6 Kohlenstoffatome enthält und die Aralkoxygruppe vorzugsweise eine Benzyloxy- oder Phenyläthyloxygruppe darstellt, bedeuten, und anschliessende Phenoletherspaltung.

Als Oxidationsmittel eignen sich gängige anorganische Oxidationsmittel, wie z.B. Kaliumpermanganat, Kaliumchromat bzw. Kaliumdichromat. Man setzt z.B. eine alkalische Permanganat-Lösung, beispielsweise eine Lösung von Kaliumpermangant und Natriumbikarbonat in Wasser, oder eine Lösung von Kaliumdichromat in einer wässerigen Säure, z.B. in Schwefelsäure, ein.

Die Oxidation erfolgt bei Temperaturen zwischen Zimmertemperatur und dem Siedepunkt des Reaktionsgemisches; mit Kaliumpermanganat beispielsweise zwischen 60 und 90 °C.

Die anschliessende Etherspaltung, also die Abspaltung des Restes $R_3$, und die Spaltung des Restes $R_{31}$, falls dieser eine Alkoxy- oder Aralkoxygruppe darstellt, erfolgt nach den im Verfahren 1 genannten Methoden; es entsteht hierbei eine Verbindung der allgemeinen Formel I.

3) Durch nucleophile Substitution des Restes $R_4$ in einem 1,2-Benzisothiazol-3(2H)-on-1,1-dioxid der allgemeinen Formel IV

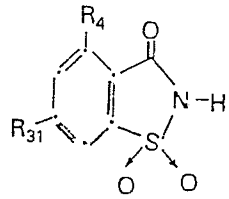

(IV) .

in der $R_{31}$ wie oben definiert ist und $R_4$ eine nucleophil ersetzbare Austrittsgruppe bedeutet und anschliessend, falls $R_{31}$ eine Alkoxy- oder Aralkoxygruppe darstellt, durch Spaltung der Phenolethergruppierung $R_{31}$. Als nucleophil ersetzbare Austrittsgruppe kommen vor allem Halogen, Pseudohalogen oder ein Diazoniumkation in Frage.

Während sich aus den Diazoniumverbindungen der allgemeinen Formel IV die Verbindungen der allgemeinen Formel I in einfacher Weise durch Erhitzen bis zum Siedepunkt in wässriger oder in saurer, wässriger Lösung, z.B. in verdünnter Schwefelsäure, herstellen lassen, gelingt der Austausch eines Halogenatoms oder eines Pseudohalogens in 4-Stellung der Formel IV in Gegenwart von Katalysatoren. Als Katalysatoren eignen sich vor allem Kupferpulver, Kupfer-(I)- oder Kupfer(II)-salze in Gegenwart von Alkalien, z.B. in Gegenwart von Natrium- oder Kaliumcarbonat. Vorzugsweise verwendet man Gemische von Kupferpulver mit Kupfersalzen, z.B. von Kupferpulver mit Kupfer(II)chlorid. Der Austausch eines Halogenatoms oder eines Pseudohalogens, wie z.B. einer Cyan- oder Rhodanidgruppe, erfolgt in Anwesenheit von Wasser bei Temperaturen zwischen 120 und 160 °C. Die Reaktion wird in einem Druckautoklaven durchgeführt. Nach der Reaktion wird das Reaktionsgemisch vom Katalysator abfiltriert, das Filtrat wird eingeengt, angesäuert und extrahiert, beispielsweise mit Ether.

Falls Substanzen der allgemeinen Formel I, in denen R eine Hydroxygruppe darstellt, erhalten werden sollen, muss noch eine Phenoletherspaltung analog den im Verfahren 1 genannten Methoden durchgeführt werden.

4) Ein 2-Sulfamoyl-3-carbonsäurederivat der allgemeinen Formel

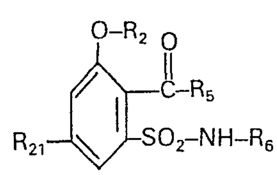

(V)

in der $R_2$ und $R_{21}$ wie oben definiert sind, $R_6$ ein Wasserstoffatom oder einen tert.-Alkylrest mit 4 bis 19 Kohlenstoffatomen und $R_5$ eine nukleophil austauschbare Gruppe, wie die Hydroxygruppe, eine Alkoxygruppe mit 1 bis 10 Kohlenstoffatomen, eine Aryloxygruppe, deren Arylrest eine Phenyl- oder Naphthylgruppe darstellt, eine Phenyl- oder Naphthylalkoxygruppe mit 1 bis 3 Kohlenstoffatomen im Alkylenteil oder ein Halogenatom bedeuten, wird bei Temperaturen zwischen 0° und 100 °C, vorzugsweise 50 bis 70 °C, mit Säuren, wie Phosphorsäure, Polyphosphorsäure, Schwefelsäure oder mit Gemischen solcher Säuren behandelt, wobei eine Cyclisierung eintritt. Das hierbei entstehende Endprodukt wird in üblicher Weise, z.B. durch Zugabe von Eis und Reinigung des dabei entstehenden Niederschlags, isoliert. Die Reaktion kann auch, in Abwesenheit einer Mineralsäure, durch blosses Erhitzen auf Temperaturen zwischen 100 und 250 °C, beispielsweise auch in Gegenwart eines Lösungsmittels, wie o-Dichlorbenzol oder Toluol, durchgeführt werden. Auch basische Reaktionsbedingungen, z.B. Umsetzung in Gegenwart von Natriummethylat, haben sich als gleich geeignet erwiesen. Ist $R_2$ in der Ausgangsverbindung eine Alkyl- oder Aralkylgruppe oder $R_{21}$ eine Alkoxy- oder Aralkoxygruppe, so wird die daraus erhaltene Verbindung noch anschliessend einer Etherspaltung unterworfen, um damit ein Produkt der allgemeinen Formel I zu erhalten. Die Etherspaltung erfolgt nach den im Verfahren 1 genannten Bedingungen.

Die Ausgangsverbindungen der allgemeinen Formel II erhält man dadurch, dass man ein, gegebenenfalls in 4-Stellung mit einer Alkoxy- oder Aralkoxygruppe substituiertes 2-Nitro-6-alkoxy (oder 6-aralkoxy-)benzonitril mit Zinn und Salzsäure in Gegenwart von Zinn(II)-chlorid durch Erhitzen am Rückfluss zum entsprechenden, gegebenenfalls in 4-Stellung mit einer Alkoxy- oder Aralkoxygruppe substituiertes 2-Amino-6-alkoxy(oder-6-aralkoxy-)benzonitril reduziert und dieses anschliessend in Gegenwart von Eisessig und Salzsäure mittels Natriumnitrit diazotiert; das Reaktionsgemisch wird hierauf in eine Lösung von Schwefeldioxid in Eisessig bei 20 °C eingetragen und eine wässrige Lösung von Kupfer(II)-chlorid langsam zugetropft. Es scheidet sich ein Sulfochlorid ab, welches in Dioxan gelöst und mit Ethanol und wässrigem konz. Ammoniak versetzt wird. Die Cyclisierung zum, gegebenenfalls in 4-Stellung mit einer Alkoxy- oder Aralkoxygruppe substituierten, 3-Amino-4-alkoxy-(oder 4-aralkoxy-)-1,2-benzisothiazol-1,1-dioxid wird durch Erwärmen auf 40 °C beendigt. Fügt man nach der letzten Stufe ein Alkalihalogenid oder -pseudohalogenid beispielsweise in Dimethylsulfoxid, z.B. Kaliumcyanid, zu, so erhält man direkt das 3-Amino-4-hydroxy-1,2-benzisothiazol-1,1-dioxid, bzw. das 3-Amino-4,6-dihydroxy- 1,2-benzisothiazol-1,1-dioxid.

Die Ausgangsverbindung der allgemeinen Formel III, in der $R_{31}$ ein Wasserstoffatom bedeutet, lässt sich aus dem entsprechenden o-Alkoxy (oder-o-Aralkoxy-)-o'-nitro-toluol gewinnen, indem man diese Verbindung, gelöst in einem Alkohol, z.B. Ethanol, mit Raney-Nickel bei 100 bar bis zur Aufnahme der theoretischen Wasserstoffmenge hydriert, wobei das entsprechende 3-Alkoxy-(oder- 3-Aralkoxy)-2-methylanilin entsteht; letztere Verbindung wird anschliessend in einer mit gasförmigem Chlorwasserstoff gesättigten, 50%igen Essigsäure gelöst und mit Natriumnitrit diazotiert. Die resultierende Diazoniumsalzlösung wird mit Magnesiumsulfat versetzt und anschliessend auf einen mit Schwefeldioxid gesättigten Eisessig gegossen, dem zuvor etwas Kupfer(I)-chlorid beigefügt wurde. Nach 4-stündigem Rühren bei Zimmertemperatur wird Wasser zugefügt und mit Methylenchlorid extrahiert. Der Extrakt wird, nach Entfernung des Extraktionsmittels, im Vakuum destilliert und das dabei rein erhaltene Sulfonsäurechlorid, gelöst in Ethanol, mit konz. wässerigem Ammoniak versetzt; hierbei entsteht das entsprechende 3-Alkoxy- oder (3-Aralkoxy)-2-methyl-benzolsulfonamid der allgemeinen Formel III. Die Darstellung der Ausgangsverbindungen der allgemeinen Formel III, in der $R_{31}$ eine Alkoxy-oder eine Aralkoxygruppe bedeutet, wird analog aus den entsprechenden p-Alkoxy (oder-p-Aralkoxy)-o-alkoxy (oder-o-aralkoxy) -o'-nitro-toluolen durchgeführt.

Die Ausgangsverbindungen der allgemeinen Formel IV erhält man beispielsweise aus dem in 6-Stellung durch einen nucleophil austauschbaren Rest (beispielsweise durch ein Chloratom) substituierten 2-Nitro-toluol durch Hydrierung desselben mittels Raney-Nickel in Gegenwart eines Lösungsmittels, wie z.B. Alkohol. Dabei entsteht das entsprechende, in 3-Stellung substituierte 2-Methyl-anilin, welches anschliessend in konz. Salzsäure mittels Natriumnitrit bei − 7° bis − 3 °C diazotiert wird. Das Filtrat wird in einem dünnen Strahl zu einer Suspension von Kupfer(II)chlorid in einer 30%igen Lösung von Schwefeldioxid in Eisessig gegossen. Es tritt Erwärmung auf ca. 40 °C ein unter starker Gasentwicklung. Man rührt noch ca. 1 Stunde bei 20 °C und eine weitere Stunde bei 35 °C und versetzt mit Eis unter Rühren. Das erhaltend, in 3-Stellung entsprechend substituierte 2-Methylbenzolsulfonsäurechlorid wird anschliessend in Dioxan gelöst und mit einer ethanolischen Ammoniaklösung bei Temperaturen zwischen 50 ° und 80 °C zum entsprechend in 3-Stellung substituierten 2-Methyl-benzolsulfonamid umgesetzt. Dieses Sulfonamid wird zusammen mit Kaliumkarbonat und Kaliumpermanganat in Wasser 1 Stunde am Rückfluss erhitzt. Das Reaktionsgemisch wird heiss filtriert und der Filterrückstand mit heissem Wasser gewaschen. Die vereinigten Filtrate werden mit Salzsäure angesäuert und gekühlt. Man erhält das entsprechend in 4-Stellung substituierte 1,2-Benzisothiazol-3(2H)-on-1,1-dioxid der allgemeinen Formel IV.

Die Ausgangsverbindungen der allgemeinen Formel V lassen sich beispielsweise wie folgt herstellen: ein m-Alkoxy-(oder Aralkoxy)-anilin, gelöst in einem polaren Lösungsmittel, z.B. in Eisessig, wird durch Zugabe von Salzsäure und Natriumnitrit diazotiert, das Reaktionsgemisch giesst

man langsam in eine Mischung aus Kupfer(II)chlorid und Kupfer(I)chlorid in einer 30%igen Lösung von Schwefeldioxid und Eisessig; die Reaktionstemperatur steigt, unter Gasentwicklung, auf ca. 40 °C, man erwärmt noch 1 Stunde auf diese Temperatur und verdünnt anschliessend mit Eiswasser. Aus der organischen Phase wird das in 3-Stellung entsprechend substituierte Benzolsulfonsäurechlorid isoliert, in Tetrahydrofuran gelöst und mit tert.-Butylamin umgesetzt (ca. 7 Stunden bei 60 °C). Aus dem Reaktionsgemisch wird das N-tert.-Butyl-3-alkoxy(oder 3-Aralkoxy)-benzolsulfonamid isoliert. Andere Reste $R_6$ lassen sich durch die Umsetzung mit anderen Aminen der Formel $R_6$-$NH_2$ analog herstellen. Die so gewonnenen Benzolsulfonamide werden anschliessend in wasserfreiem Tetrahydrofuran gelöst und bei −80 bis −50 °C mit Lithiumdiisopropylamid oder n-Butyllithium umgesetzt. Anschliessend wird das Reaktionsgemisch bei 0 °C noch ca. 1 Stunde gerührt, auf −70 bis −50 °C abgekühlt und mit Kohlendioxidgas behandelt. Man lässt bei Zimmertemperatur zu Ende reagieren und isoliert aus dem Reaktionsgemisch die 2-(N-tert.Butyl)-sulfamoyl-6-(alkoxy oder aralkoxy)-benzoesäure der allgemeinen Formel V, wobei in diesem Fall $R_5$ die Hydroxygruppe und $R_6$ die tert. Butylgruppe bedeuten. Bei Verwendung anderer Amine der Formel $R_6$-$NH_2$ enthält man die entsprechenden, in 2-Stellung substituierten Sulfamoylbenzoesäuren; diese Benzoesäuren lassen sich in an sich bekannter Weise anschliessend verestern.

Wie eingangs erwähnt, besitzen die Verbindungen der allgemeinen Formel I und deren physiologisch verträglichen Salze Süssstoffeigenschaften.

Es wurden beispielsweise

| | |
|---|---|
| 4-Hydroxy-1,2-benzisothiazol-3(2H)-on-1,1-dioxid | = A |
| 4-Hydroxy-1,2-benzisothiazol-3(2H)-on-1,1-dioxidnatriumsalz | = B |
| 4,6-Dihydroxy-1,2-benzisothiazol-3(2H)-on-1,1-dioxid | = C |
| im Vergleich zu den bekannten Süssstoffen Cyclamat und | = X |
| Saccharin | = Y |
| und im Vergleich zu der literaturbekannten Verbindung 6-Hydroxy-1,2-benzisothiazol-3(2H)-on-1,1-dioxid | = Z |

untersucht.

A Bestimmung der Süssstoffeigenschaften

1) Bestimmung der Grenzkonzentration (threshold concentration)

Es wurden von den Substanzen A, B und C und von den Vergleichsverbindungen X, Y und Z wässrige Lösungen in den Konzentrationen 1:12 500, 1:25 000, 1:50 000, 1:100 000 und 1:200 000 hergestellt und von jeweils 5 Geschmackstestern nach den folgenden Kriterien: sehr süss (= 3 Punkte), mittelmässig süss (= 2 Punkte), schwach süss (= 1 Punkt) und nicht süss (= 0 Punkt) bewertet. Die weitere Durchführung des Tests erfolgte nach den Angaben von H.G. Schutz und F.J. Pilgrim [Food Research 22, 206 (1957)]. Die als süss wahrnehmbare Verdünnung ist als die Konzentration definiert, bei der der Mittelwert die Bewertungs-Punkte 1,0 oder mehr erreicht.

In der nachfolgenden Tabelle 1 sind die erzielten Ergebnisse zusammengefasst:

Tabelle 1

| Substanz | Konzentration | Mittelwert der Bewertungspunkte | Als süss wahrnehmbare Verdünnung |
|---|---|---|---|
| A | 1:25 000 | 2,4 | 1:50 000 |
| | 1:50 000 | 1,0 | |
| | 1:100 000 | 0,2 | |
| B | 1:25 000 | 2,6 | 1:50 000 |
| | 1:50 000 | 1,2 | |
| | 1:100 000 | 0,0 | |
| C | 1:25 000 | 1,8 | 1:50 000 |
| | 1:50 000 | 1,0 | |
| | 1:100 000 | 0,0 | |
| Vergleichssubstanzen: | | | |
| X | 1:12 500 | 0,6 | >1:12 500 |
| | 1:25 000 | 0,2 | |
| | 1:50 000 | 0,0 | |
| Y | 1:25 000 | 2,4 | 1:50 000 |
| | 1:50 000 | 1,2 | |
| | 1:100 000 | 0,4 | |
| Z | 1:25 000 | 1,4 | 1:25 000 |
| | 1:50 000 | 0,6 | |
| | 1:100 000 | 0,0 | |

2) Bestimmung der relativen Süsskraft im Vergleich zu Saccharose

Die relative Süsskraft (häufig auch als Süssungsgrad definiert) von Süssstoffen im Vergleich zu Saccharose (Rohrzucker) ändert sich mit den Konzentrationen in weiten Grenzen. So schwankt in den gebräuchlichen Konzentrationen (entsprechend einer 2–10%igen Saccharoselösung) die relative Süsskraft des Saccharins zwischen 200 und 700. Es wurde daher bei der Bestimmung der relativen Süsskraft der obengenannten Verbindungen stets eine 3%ige wässrige Saccharoselösung als Vergleich herangezogen.

Die weitere Durchführung der Bestimmung der relativen Süsskraft erfolgte weitgehend nach den Angaben von R. Pauli [Chemiker-Zeitung 44, 744 (1920)] und T. Paul [Chemiker-Zeitung 45, 38 (1921)]. Jede Untersuchungsreihe wurde von 5 Geschmackstestern untersucht.

Die erzielten Ergebnisse mit den Substanzen A, B und C und mit den Vergleichssubstanzen X und Z sind in der nachfolgenden Tabelle 2 zusammengefasst:

Tabelle 2

| Substanz | Relative Süsskraft Saccharose = 1 |
|---|---|
| A | 210 |
| B | 210 |
| C | 180 |
| Vergleichssubstanz | |
| X | 45 |
| Z | 140 |

3) Bestimmung der Geschmacksqualität

Neben der absoluten Süsskraft kommt der Geschmacksqualität eines Süssstoffes eine grosse Bedeutung zu. So übertreffen Saccharin und Cyclamat die Saccharose in ihrer absoluten Süsskraft um ein Vielfaches, sie erreichen aber bei weitem nicht die Geschmacksqualitäten der Saccharose. So lässt sich der bekannte metallischbittere Nachgeschmack und der bittere Beigeschmack des Saccharins bei Geschmacksprüfungen klar demonstrieren. Da eine der Hauptforderungen an einen Süssstoff ein der Saccharose vergleichbarer Geschmack ist, wurde eine Bestimmung der Geschmacksqualitäten durchgeführt.

Die Testung erfolgte in Anlehnung an die Methode von DuBois, G.A. Crosby, R.A. Stephanson und R.E. Wingard Jr., [J. Agric. Food Chem. 25, 763 (1977)]. Die Substanzproben wurden als wässrige Lösungen in einer Konzentration, die ca. einer 5%igen Saccharoselösung entsprechen, verwendet. Als Vergleich diente Saccharose. Die Versuche wurden unter identischen äusseren Bedingungen durchgeführt. Es wurde nur die Charakterisierung des Geschmacks beurteilt, da die Intensität bereits in vorangegangenen Experimenten bestimmt worden war. Es wurden die folgenden Kriterien von jeder Testperson bestimmt: Prozentsatz süss zu Prozentsatz bitter zu Prozentsatz sonstige Geschmacksqualität und die Anwesenheit oder Abwesenheit eines Nachgeschmacks der einzelnen Proben. Es wurden zweimal fünf Bestimmungen für jede Substanz durchgeführt und die Ergebnisse sind in der nachfolgenden Tabelle 3 aufgeführt.

Tabelle 3

| Substanz | Konzentration | Zahl der Bestimmungen | Prozentsatz der Anteile süss | bitter | sonstig | Nachgeschmack |
|---|---|---|---|---|---|---|
| A | 1:10 000 | 10 | 94 | 2 | 4 | 3 |
| B | 1:10 000 | 10 | 96 | 2 | 2 | 5 |
| C | 1:10 000 | 10 | 86 | 3 | 11 | 8 |
| Vergleichssubstanzen | | | | | | |
| Y | 1:10 000 | 10 | 72 | 21 | 7 | 80 |
| Z | 1:10 000 | 10 | 81 | 7 | 12 | 29 |
| Saccharose | 1:20 | 10 | 98 | 0 | 2 | 4 |

Aus der Tabelle kann entnommen werden: Das Idealprofil der Saccharose wird von den Vergleichssubstanzen Y und Z bei weitem nicht erreicht. Dagegen zeigen die Substanzen A bis C eine weitgehende Freiheit an Nachgeschmack, was für Süssstoffe überraschend ist. Ebenso ist die Reinheit des Geschmacks (94–96%) für die Substanzen A und B und 86% für die Substanz C für Süssstoffe ungewöhnlich gut.

4) Bestimmung der Löslichkeit und der Säurestabilität

Eine weitere Anforderung an einen Süssstoff ist eine gute Wasserlöslichkeit und eine Stabilität in saurem Medium. Orientierend wurde festgestellt, dass die Substanz B in Wasser leicht löslich ist (Löslichkeit>> 1 g/10 ml Wasser).

Bei einer orientierenden Stabilitätsuntersuchung wurde die Substanz B in einer Konzentration von 1% in 0,1 N Salzsäure gelöst und 27 Tage bei Raumtemperatur stehengelassen. Es trat keinerlei Veränderung ein. Ausserdem wurde die Festsubstanz im Tageslicht und bei 60 °C mit und ohne Lichtausschluss über 4 Wochen geprüft. Es trat ebenfalls keine Veränderung ein.

B. Prüfung auf etwaige pharmakologische Wirkungen

Es wurde die Substanz B eingehend auf etwaige pharmakologische und toxische Wirkungen untersucht. Im nachfolgenden werden die einzelnen Untersuchungen und ihre Ergebnisse kurz geschildert:

1. Sedierende oder erregende Wirkung:

Beeinflussung der Motilität von Mäusen

Methode:

Acht weibliche, 20–26 g schwere Chbb:NMRI-Mäuse (SPF) wurden nach oraler Substanzgabe in einer Suspension 1%iger Tylose einzeln in Lichtschrankenaktivitätskäfige gesetzt und ihre Motilität 30, 90 und 150 min nach Substanzgabe für jeweils 5 min gemessen und als Prozent der Motilität einer Suspensionsmittel-Kontrollgruppe bewertet (Tylose:Carboxymethylcellulose).

Ergebnis:
Tabelle 1: Motilität von Mäusen, Kontakte/Tier ± SD

| | | min nach Applikation | | |
|---|---|---|---|---|
| Behandlung | N | 30–35 | 90–95 | 150–155 |
| Kontrolle | 8 | 90±35 | 83±22 | 68±23 |
| Substanz B 200 mg/kg p.o. | 8 | 92±21 | 84±28 | 49±17 |
| Substanz B % Motilität | | 102 | 101 | 72 |

N = Anzahl der Tiere

Die Substanz B beeinflusste in der grossen Dosis von 200 mg/kg p.o. die Motilität von Mäusen nicht.

2. Sedierende oder erregende Wirkung:
Beeinflussung der Motilität von Ratten
Methode:

In Anlehnung an FÜHRER und FELDHOFEN (Arzneim.-Forsch. 11, 1027, 1961) wurde die Substanz B mit 100 mg/kg p.o. in einer Suspension 1%iger Tylose 6 weiblichen, 160–190 g schweren Chbb:THOM-Ratten (SPF) verabreicht, die Motilität der einzeln in den Testkäfigen befindlichen Tiere ab 1 h nach Substanzgabe für 30 min Dauer gemessen und in Prozent der Motilität einer Tylosekontrollgruppe bewertet.

Ergebnis:
Tabelle 2: Motilität von Ratten, Kontakte/Tier ± SD

| Behandlung | N | min nach Applikation 60–90 |
|---|---|---|
| Kontrolle | 6 | 208±49 |
| Substanz B 100 mg/kg p.o. | 6 | 235±58 |
| Substanz B % Motilität | | 113 |

N = Anzahl der Tiere

Die Substanz B beeinflusste mit 100 mg/kg p.o. die Motilität von Ratten nicht.

3. Muskelrelaxierende und/oder Ataxie-Wirkung:
Beeinflussung des Haltevermögens von Mäusen in rotierenden Drahtzylindern

Methode:

In Anlehnung von YOUNG und LEWIS (Science 105, 368, 1947) wurden 10 weibliche, 20–26 g schwere Chbb:NMRI-Mäuse (SPF) nach oraler Gabe von 400 mg/kg Substanz in Aqua dest.-Lösung zu den Zeiten 30–60, 90–120, 210–240 und 270–300 min in langsam rotierenden, schräggestellten Drahtzylindern auf ihr Haltevermögen geprüft.

Ergebnis:
Tabelle 3: Beeinflussung des Haltevermögens von Mäusen

| | | herausgefallene Tiere, min nach Applikation | | | |
|---|---|---|---|---|---|
| Behandlung | N | 30–60 | 90–120 | 210–240 | 270–300 |
| Substanz B 400 mg/kg p.o. | 10 | 2 | 2 | 1 | 0 |

N = Anzahl der Tiere

Substanz B hatte selbst in der sehr grossen Dosis 400 mg/kg p.o. praktisch keinen Einfluss auf das Festhaltevermögen von Mäusen. Die Substanz wirkte nicht muskelrelaxierend und/oder koordinationshemmend.

4. Barbituratverstärkende Wirkung:
Beeinflussung der Dauer der Hexobarbitalnarkose bei Mäusen
Methode:

Nach WINTER (J. Pharmacol. exp. Ther. 94, 7, 1948) erhielten 10 männliche, 20–26 g schwere Chbb:NMRI-Mäuse (SPF) 100 mg/kg der zu prüfenden Substanz B in einer Suspension 1%iger Tylose p.o.. Eine Kontrollgruppe erhielt das Suspensionsmittel. 1 h später wurde allen Tieren 80 mg/kg Hexobarbital i.p. injiziert. Die Mäuse wurden auf eine 37 °C warme Metallplatte gelegt und die Zeit vom Beginn des Stellreflexverlustes bis zu ihrem Wiederauftreten als Narkosedauer gemessen. Diese wurde in Prozent der Suspensionsmittelkontrollen bewertet.

Ergebnis:
Tabelle 4: Beeinflussung der Dauer der Hexobarbitalnarkose bei Mäusen

| Behandlung | Narkosedauer min±SD | % |
|---|---|---|
| Substanz B 100 mg/kg p.o. | 15,65±6,32 | 106 |
| Kontrolle | 14,70±3,62 | 100 |

Die Substanz B hatte mit 100 mg/kg p.o. bei Mäusen keinen Einfluss auf die Dauer der Hexobarbitalnarkose.

5. Antikonvulsive Wirkung:
Beeinflussung des maximalen Elektrokrampfes bei Mäusen
Methode:

Nach SWINYARD, BROWN und GOODMAN (J. Pharmacol. exp. Ther. 106, 319, 1952) wurde die Substanz B 10 männlichen, 20–26 g schweren Chbb:NMRI-Mäusen (SPF) in Aqua dest.-Lösung mit 200 mg/kg oral verabreicht. 30, 150 und 300 min später wurden bei den Tieren tonische Extensorkrämpfe durch Wechselstrom (50 mA, 50 Hz, 2 sec Dauer) über Kopfelektroden ausgelöst. Antikonvulsiv wirkende Substanzen lassen die tonischen Extensorkrämpfe der Extremitäten nicht auftreten.

Ergebnis:
Tabelle 5: Beeinflussung des maximalen Elektrokrampfes bei Mäusen

| Behandlung | N | Zahl der geschützten Tiere 30 | 150 | 300 min nach Substanz |
|---|---|---|---|---|
| Substanz B 200 mg/kg p.o. | 10 | 0 | 0 | 0 |

N = Anzahl der Tiere

Die Substanz B hatte mit 200 mg/kg p.o. bei Mäusen keine antikonvulsive Wirkung.

6. Antidepressive Wirkung:
Einfluss auf die Reserpin-Hypothermie bei Mäusen
Methode:

In Anlehnung an ASKEW (Life Sci. 2, 275, 1963) wurde bei männlichen, 20–26 g schweren Chbb:NMRI-Mäusen (SPF) durch 3 mg/kg Reserpin s.c. eine Hypothermie erzeugt. 17 h später wurde bei allen Tieren die erniedrigte Körpertemperatur gemessen und anschliessend die Substanz B mit 40 und 100 mg/kg in Aqua dest.-Lösung an 10 Tiere/Dosis oral verabreicht. Eine Kontrollgruppe erhielt das Lösungsmittel p.o.. 1 bis 4 h (Stunden) nach Substanzgabe wurde stündlich die Temperatur gemessen. Die in der Tabelle aufgeführten Werte sind Temperatur-Mittelwerte ± SD.

Ergebnis:
Tabelle 6: Verhalten der Körpertemperatur im Vergleich zu den Kontrollen; °C ± SD

| Behandlung | N | vor Substanzgabe | h nach Substanzgabe 1 | 2 | 3 | 4 |
|---|---|---|---|---|---|---|
| Kontrolle | 10 | 22,6 ±0,2 | 24,6 ±1,3 | 25,7 ±1,9 | 26,2 ±2,1 | 26,5 ±1,9 |
| Substanz B 40 mg/kg p.o. | 10 | 22,7 ±0,3 | 24,0 ±1,4 | 24,7 ±1,7 | 25,3 ±1,7 | 25,9 ±1,4 |
| Substanz B 100 mg/kg p.o. | 10 | 23,3 ±0,5 | 26,0 ±1,7 | 26,7 ±1,4 | 26,6 ±1,2 | 26,8 ±1,4 |

N = Anzahl der Tiere

Die Substanz B hatte mit 40 und 100 mg/kg p.o. praktisch keinen Einfluss auf die Reserpin-Hypothermie bei Mäusen.

7. Antiexsudative Wirkung:
Einfluss auf das Kaolinödem bei Ratten
Methode:

Nach HILLEBRECHT (Arzneim.-Forsch. 4, 607, 1954) wurde bei 125–150 g schweren Chbb:THOM-Ratten (SPF) beiderlei Geschlechts durch subplantare Injektion von 0,05 ml einer 10%igen Kaolinsuspension in eine Hinterpfote ein Ödem ausgelöst. 30 min vorher wurde die Substanz B mit 100 mg/kg als Aqua dest.-Lösung an 10 Versuchstiere oral verabreicht. 10 Kontrolltiere erhielten das Lösungsmittel. Vor Substanzgabe und 5 h danach wurden die Pfotendicken gemessen. Die Ödem-verändernde Wirkung wurde in Prozent des mittleren Schwellungsgrades der Kontrolltiere bewertet.

Ergebnis:
Tabelle 7: Wirkung gegen das Kaolinödem bei Ratten

| Behandlung | N | mittlerer Schwellungswert | Hemmung in % |
|---|---|---|---|
| Substanz B 100 mg/kg p.o. | 10 | 239,0 | 13,7 |
| Kontrolle | 10 | 277,0 | |

N = Anzahl der Tiere

Die Substanz B hatte mit 100 mg/kg p.o. bei Ratten praktisch keine antiexsudative Wirkung.

8. Ulcerogene Wirkung bei Ratten
Methode:

Zehn weiblichen und männlichen (1:1), um 200 g schweren Chbb: THOM-Ratten (SPF) wurde die Substanz B mit 200 mg/kg p.o. in 1%iger Tylosesuspension oral verabreicht. Eine Kontrollgruppe erhielt das Suspensionsmittel. 4 h später wurden alle Tiere abgetötet, der Magen freipräpariert und auf Schleimhautläsionen inspiziert.

Ergebnis:
Tabelle 8: Ulcerogene Wirkung bei Ratten

| Behandlung | N | Tiere mit Magenläsionen |
|---|---|---|
| Substanz B 200 mg/kg p.o. | 10 | 0 |
| Kontrolle | 10 | 0 |

N = Anzahl der Tiere

Die Substanz B hatte mit 200 mg/kg p.o. bei Ratten keine ulcerogene Wirkung.

9. Einfluss auf die Cholerese bei Ratten
Methode:

Bei nüchternen, männlichen, 180–220 g schweren Chbb:THOM-Ratten (SPF) wurde in Urethannarkose eine Kanüle in den Ductus choledochus gebunden und die Gallenflüssigkeit über Tropfenzähler registriert. Jeweils 10 Tieren wurde die Substanz B mit 100 oder 200 mg/kg in Aqua dest.-Lösung intraduodenal appliziert. Eine Kontrollgruppe erhielt das Lösungsmittel. Die Anzahl der Tropfen während 1 h vor Behandlung wurde mit der 1–5 h nach Behandlung verglichen.

Ergebnis:
Tabelle 9: Cholerese bei Ratten, mittlere Tropfenzahl/h ± SD

| | | | h nach Behandlung | | | | |
|---|---|---|---|---|---|---|---|
| Behandlung | N | Vorwert | 1 | 2 | 3 | 4 | 5 |
| Kontrolle | 10 | 62,6 ±4,8 | 57,1 ±4,8 | 55,8 ±5,1 | 56,3 ±6,0 | 56,6 ±6,9 | 58,1 ±10,0 |
| Substanz B 100 mg/kg i.d. | 10 | 55,1 ±9,1 | 53,3 ±10,1 | 51,7 ±9,2 | 50,7 ±9,3 | 49,9 ±9,2 | 49,5 ±9,1 |
| Substanz B 200 mg/kg i.d. | 10 | 47,1 ±11,5 | 44,1 ±9,8 | 43,1 ±10,3 | 43,1 ±8,2 | 43,5 ±8,2 | 44,4 ±8,0 |

N = Anzahl der Tiere

In Tabelle 10 wurde die mittlere Tropfenzahl 1–5 h nach Behandlung in Prozent des jeweiligen Vorwertes dargestellt.

Tabelle 10: Cholerese bei Ratten in Prozent des Wertes vor Behandlung

| Behandlung | N | Vorwert | h nach Behandlung 1 | 2 | 3 | 4 | 5 |
|---|---|---|---|---|---|---|---|
| Kontrolle | 10 | 100 | 91 | 89 | 90 | 90 | 93 |
| Substanz B 100 mg/kg i.d. | 10 | 100 | 97 | 94 | 92 | 91 | 90 |
| Substanz B 200 mg/kg i.d. | 10 | 100 | 94 | 92 | 92 | 92 | 94 |

N = Anzahl der Tiere

Wie die Tabellen 9 und 10 zeigen, hatte die Substanz B mit 100 und 200 mg/kg intraduodenal keinen Einfluss auf die Cholerese bei Ratten.

10. Kreislaufwirkung:
Einfluss auf den Kreislauf narkotisierter Katzen
Methode:
Bei 3 adulten Katzen in Nembutalnarkose wurden folgende Parameter gemessen:
der arterielle Blutdruck in der Arteria carotis mittels Statham-Druckumwandler, die Herzfrequenz mittels eines Grass-Tachographen, die Atemfrequenz mittels eines Fleisch'schen Pneumotachographen.
Die Substanz B wurde als Lösung in Aqua dest. mit 10 mg/kg i.v. verabreicht.

Ergebnis:
Tabelle 11: Blutdruck, Herzfrequenz und Atemfrequenz narkotisierter Katzen, prozentuale Veränderung gegenüber Werten vor Substanzgabe

| Behandlung | N | Prozentuale Veränderungen: Blutdruck | Herzfrequenz | Atemfrequenz |
|---|---|---|---|---|
| Substanz B 10 mg/kg i.v. | 3 | +1 | −1 | −2 |

N = Anzahl der Tiere

Die Substanz B hatte mit 10 mg/kg i.v. keinen Einfluss auf den arteriellen Blutdruck, die Herzfrequenz und die Atemfrequenz narkotisierter Katzen.

11. Einfluss auf die Thrombozytenaggregation
Methode:
Die Thrombozytenaggregation wurde nach BORN und CROSS (J. Physiol. 170, 397, 1964) in durch Zentrifugieren menschlichen Citratblutes gewonnenem plättchenreichen Plasma gemessen. Parameter waren die maximale Aggregationsgeschwindigkeit in Prozent Veränderung gegenüber einer Kontrolle und die prozentuale Veränderung der «optical density».

Die Aggregation wurde durch Collagen ausgelöst.

Ergebnis:
Tabelle 12: Wirkung auf die Collagen-bedingte Thrombozytenaggregation

| Behandlung | Veränderung in %: max. Aggregationsgeschwindigkeit | optical density |
|---|---|---|
| Substanz B $10^{-4}$ Mol/l | ±0 | ±0 |

Die Substanz B hatte mit $1 \times 10^{-4}$ Mol/l keinen Einfluss auf die Collagen-bedingte Thrombozytenaggregation.

12. Bacteriostase und Fungistase
Methoden:
Die Substanz B wurde im Reihenverdünnungstest und im Agardiffusionstest auf bakterien- und fungihemmende Wirkung geprüft.

Ergebnis:
Im Reihenverdünnungstest hatte Substanz B mit 320 µg/ml keine Wirkung auf Staphylococcus aureus SG 511, Streptococcus Aronson, Sc. pyogenes ATCC 8668, Escherichia coli ATCC 9637, Pseudomonas aeruginosa Hbg., Serratia marcescens ATCC 13880, Candida albicans ATCC 10231, Trychophyton mentagrophytes ATCC 9129 und Aspergillus niger.

Auch im Agardiffusionstest war Substanz B mit 16 000 µg/ml gegen die gleichen Keime unwirksam.

13. Diuretische Wirkung:
Einfluss auf die Diurese von Ratten
Methode:
Männlichen, 180–210 g schweren Chbb:THOM-Ratten (SPF) wurde bei Zugang zu Wasser 24 h vor dem Versuch die Nahrung entzogen. Substanz B wurde 6 Tieren/Dosis in den Dosierungen 50, 100 und 200 mg/kg in Aqua dest.-Lösung mit 1 ml/100 g Körpergewicht oral verabreicht. Zusätzlich

wurden 4 ml/100 g Körpergewicht oral verabreicht. Zusätzlich wurden 4 ml/100 g Körpergewicht Wasser oral gegeben. Eine Kontrollgruppe erhielt das Lösungsmittel und die Wasserbelastung oral.

5 h nach Behandlung wurde für jede Behandlungsgruppe das Harnvolumen gemessen und die Konzentration von $Na^+$, $K^+$ und $Cl^-$ photometrisch und komplexometrisch bestimmt.

Ergebnis:
Tabelle 13: Diurese bei wasserbelasteten Ratten

| Behandlung | N | Microval/kg $Na^+$ | $K^+$ | $Cl^-$ | Harn ml/kg |
|---|---|---|---|---|---|
| Substanz B | | | | | |
| 50 mg/kg p.o. | 6 | 456,6 | 456,6 | 616,4 | 45,7 |
| 100 do. | 6 | 401,9 | 321,5 | 325,5 | 40,2 |
| 200 do. | 6 | 467,6 | 425,1 | 535,7 | 42,5 |
| Kontrolle | 6 | 274,9 | 471,2 | 353,4 | 39,3 |

N = Anzahl der Tiere

Die Substanz B hatte mit 50, 100 und 200 mg/kg p.o. keinen Einfluss auf die Diurese bei Ratten. Alle gefundenen Ausscheidungswerte liegen im Vertrauensbereich ($p \leq 0,05$) von 50 Kontrollmittelwerten:
$Na^+$ : 154,3 – 660,0 µVal/kg
$K^+$ : 146,3 – 550,0 µVal/kg
$Cl^-$ : 189,9 – 673,1 µVal/kg
Volumen: 37,1 – 49,3 ml/kg

14. Schleimhautverträglichkeit:
Lokale Verträglichkeit am Kaninchenauge
Methode:
Jeweils 4 Kaninchen (♀, ♂) wurde 1 Tropfen einer 1%igen Lösung von Substanz B in Aqua dest. in den Conjunktivalsack eines Auges eingetropft und die Augen zu den Zeiten 15, 30, 60, 90, 120 min sowie 24 h danach auf Conjunktivareizung (Rötung, Schleimabsonderung etc.) sowie auf Pupillenveränderung beobachtet. Das 2. Auge jedes Tieres diente als Kontrolle.

Ergebnis:
Tabelle 14: Lokale Verträglichkeit, Kaninchenauge

| Behandlung | N | Befund |
|---|---|---|
| Substanz B | | |
| 1%ig | 4 | keine Conjunktivareizung, keine Pupillenveränderung |
| 10%ig | 4 | keine Conjunktivareizung, keine Pupillenveränderung |

N = Anzahl der Tiere

Die Substanz B wurde von Kaninchen bei Eintropfen von Substanzlösung in das Auge gut vertragen.

C. Prüfung auf etwaige akute, subakute, toxische und mutagene Wirkungen

1. Prüfung auf akute Toxizität
Die akute Toxizität der Substanz B wurde nach oraler Gabe an männlichen und weiblichen Mäusen, Ratten und Hunden bestimmt. Die Substanz wurde als Suspension in Tylose im Falle der Applikation an Mäusen und Ratten und als Lösung in Wasser im Fall der Applikation an Hunden verabreicht. In der nachfolgenden Tabelle sind die nach der angegebenen Dosierung innerhalb von 1,7 und 14 Tagen gestorbenen Tiere angegeben.

| Tierart | Dosis | Tierzahl | Gestorbene Tiere in der Beobachtungszeit 1 Tag | 7 Tage | 14 Tage |
|---|---|---|---|---|---|
| Maus | 10 g/kg | 10 | 0 | 0 | 0 |
| Ratte | 10 g/kg | 6 | 0 | 0 | 0 |
| Hunde | 5 g/kg | 2 | 0 | 0 | 0 |

In der Beobachtungszeit sind nicht nur keine Tiere gestorben, sondern es konnten an den Tieren keinerlei toxische Symptome beobachtet werden. Alle Tiere überlebten mit völlig normalem Verhalten die überaus hohen Dosierungen von 10 g/kg.

2. Prüfung auf subakute Toxizität

Die subakute Toxizität der Substanz B wurde an Ratten und Hunden durchgeführt.

Untersuchung an der Ratte:

Es wurden 10 männliche und 10 weibliche Ratten des Stammes Chbb:THOM in den Versuch genommen. Über einen Zeitraum von 5 Wochen erhielten die Tiere täglich 750 mg/kg der Substanz B verabreicht. Während des angesetzten Versuchszeitraumes verhielten sich die Tiere unauffällig. Spontantodesfälle traten nicht auf. Nach Ablauf der 5 Behandlungswochen wurden die Tiere getötet und folgende Organe für eine histologische Untersuchung präpariert: Herz, Lunge, Leber, Milz, Nebennieren, Gehirn, Niere und Bauchspeicheldrüse.

Die histologische Untersuchung dieser Organe ergab keinerlei pathologischen Befunde, die mit der Substanzgabe in Zusammenhang zu bringen wären.

Untersuchung am Hund:

Von dieser Spezies wurden je 3 männliche und weibliche Beagle-Hunde in den Versuch genommen. Diese Tiere erhielten gleichfalls über einen Zeitraum von 5 Wochen die Substanz B in einer Dosierung von 750 mg/kg in der oralen Form. Es wurden keine Störungen im Allgemeinbefinden beobachtet. Spontantodesfälle traten nicht auf.

Nach Ablauf der angesetzten Versuchszeit von 5 Wochen wurden die Tiere getötet und die gleichen Organe wie bei der Ratte für eine histologische Untersuchung asserviert und präpariert.

An den genannten Organen konnten keine Befunde aufgedeckt werden, die mit der Substanzgabe in Verbindung hätten gebracht werden können.

3. Prüfung auf mutagene Wirkung

Die wichtigste Forderung an einen Süssstoff ist die gesundheitliche Unbedenklichkeit dieser Substanzen. Diese Forderung erfüllten jedoch die meisten Süssstoffe nicht. So zeigt zum Beispiel das Saccharin eine mutagene Wirkung (R.P. Batzinger, S.-Y.L. On und E. Bueding. Science 198, 944 (1977)), die seine Verwendung als Süssstoff in Frage stellt.

Die Substanz B wurde im ANES-System [Mutation Research 31, 347–364 (1975)] auf ihre mutagenen Eigenschaften untersucht. Um das gesamte Spektrum induzierter molekularer DNA-Schäden (Basensubstitutionen, Rasterschubmutationen, Deletionen) nachweisen zu können, wurden als genetische Indikatoren die auxotrophen Bakterienstämme Salmonella typhimurium TA 98, TA 100, TA 1535, TA 1537 und TA 1538 eingesetzt.

Für alle Keime waren die Tests so aufgebaut, dass der Einfluss eines mikrosomalen Enzymsystems mit den entsprechenden Cofaktoren auf die Prüfsubstanz und Unterschiede in der Enzyminduktion untersucht werden konnten. Die Testung erfolgte ohne und mit Zugabe eines Aktivierungssystems. Für die in vitro-Aktivierung wurden S-9 Überstände (Bruchstücke des endoplasmatischen Retikulums) von normalen, d.h. nicht vorbehandelten Ratten und von mit Aroclor 1254 (500 mg/kg) induzierten Ratten verwendet. Von der induzierten S-9 Fraktion wurden 3 verschiedene Konzentrationsstufen geprüft.

Die Substanzkonzentrationen lagen bei 0,25, 2,5, 5 und 10 mg/Platte. In einem vorausgehenden Reihenverdünnungstest zeigte die höchste Dosierung noch keine Hemmung des Keimwachstums.

In keinem der untersuchten Stämme führte die Substanz B zu einem Anstieg der spontanen Mutationsrate. Ein Einfluss der Enzyminduktion und der Aktivierungsfraktion auf die Rückmutationsereignisse war nicht nachweisbar. Die gezählten Kolonien auf den Substanzplatten lagen im Spontanbereich für die jeweiligen Teststämme.

Die Experimente zeigen, dass die Substanz B keine Basensubstitutionen [S. typhimurium TA 1535, TA 100] und keine Frameshiftmutationen [S. typhimurium TA 1537, TA 1538, TA 98] auslösen, und ergeben somit keinen Hinweis für eine mutagene Potenz der Substanz B.

Die nachfolgenden Beispiele sollen die Herstellung der Süssstoffe verdeutlichen:

Beispiel 1

4-Hydroxy-1,2-benzisothiazol-3(2H)-on-1,1-dioxid

5 g (25,2 mmol) 3-Amino-4-hydroxy-1,2-benzisothiazol-1,1-dioxid und 2,02 g (50,5 mmol) Natriumhydroxid wurden in 15 ml Wasser 6,5 Stunden am Rückfluss erhitzt. Danach wurde die Lösung bei 60 °C mit Aktivkohle behandelt und filtriert. Das Filtrat wurde bei 60 °C mit konz. wässriger Salzsäure (ca. 5 ml, ca. 0,06 Mol) angesäuert. Der entstandene Niederschlag wurde abfiltriert, getrocknet und aus Wasser umkristallisiert. Es wurden 4,62 g (92% d. Theorie) 4-Hydroxy-1,2-benzisothiazol-3(2H)-on-1,1-dioxid erhalten; Schmelzpunkt: 228 °C.

IR($CH_2CL_2$): 3320 (NH, OH assoz.); 1720 (CO); 1335 und 1140 $cm^{-1}$ ($SO_2$)

MS: 199 (M+), 136, 119 und 108 m/e.

$C_7H_5NO_4S$ (199,19)

| | | | | |
|---|---|---|---|---|
| Ber.: | C 42,20 | H 2,53 | N 7,03 | S 16,10 |
| Gef.: | 42,23 | 2,60 | 6,97 | 16,10 |

Die Ausgangssubstanz 3-Amino-4-hydroxy-1,2-benzisothiazol-1,1-dioxid wurde auf die folgende Weise, ausgehend von 2-Nitro-6-methoxy-benzonitril (A. Russel u. W.G. Tebbens, Org. Synthesis, Coll. Vol. III, p. 293) hergestellt:

2-Amino-6-methoxy-benzonitril

10 g (56,1 mmol) 2-Nitro-6-methoxy-benzonitril, 26,6 g (220 mmol) Zinn und 12,7 g (56,1 mmol) Zinn(II)-chlorid wurden in 160 ml halbkonzentrierter Salzsäure 1,5 Stunden am Rückfluss erhitzt. Danach wurde das Reaktionsgemisch auf 60 °C abgekühlt und bei dieser Temperatur wurde eine Lösung von 40 g Natriumhydroxid in 10 ml Wasser zugetropft. Nach vollständiger Natronlauge-Zugabe (pH: ca. 4) wurde durch Zugabe von Natriumcarbonat auf pH 9 eingestellt und der entstandene Niederschlag abgesaugt. Der Filterrückstand wurde mit 0,2 l Äthanol 20 Minuten am Rückfluss erhitzt und filtriert. Das Filtrat wurde eingeengt und ergab 9 g Rohmaterial. Die Extraktion des Zinnhydroxid-Produkt-Gemisches wurde wiederholt und ergab noch ca. 0,5 g Rohmaterial.

Das Rohprodukt aus Ethanol wurde unter Verwendung von Aktivkohle umkristallisiert. Die eingeengte Mutterlauge ergab eine 2. Fraktion gleicher Reinheit. Man erhielt 7,5 g (90% der Theorie) 2-Amino-6-methoxy-benzonitril vom Schmelzpunkt 145 °C

3-Amino-4-methoxy-1,2-benzisothiazol-1,1-dioxid

10 g (67,5 mmol) 2-Amino-6-methoxy-benzonitril wurden in 80 ml Eisessig bei 60 °C gelöst und mit 80 ml konz. wässriger Salzsäure in einem Guss versetzt. Danach wurde das Reaktionsgemisch auf − 5 °C gekühlt und bei dieser Temperatur durch Zutropfen von einer Lösung von 4 g Natriumnitrit in 18,5 ml Wasser diazotiert. Das Reaktionsgemisch wurde 1/2 Stunde nachgerührt (Temperaturanstieg auf ca. 0 °C) und in eine vorbereitete Mischung aus 94 ml einer 30%igen Schwefeldioxidlösung in Eisessig und einer Lösung von 1,7 g Kupfer(II)-chlorid in 4 ml Wasser zügig portionsweise eingetragen. Die Temperatur der vorgelegten Mischung betrug 20 °C und diese Temperatur wurde auch während des Eintragens der Diazoniumsalzlösung beibehalten.

Anschliessend wurde das Reaktionsgemisch 3 Stunden nachgerührt, wobei die Farbe von rot nach gelbgrün umschlug und ein Teil des Sulfochlorides sich abschied. Das Sulfochlorid wurde abfiltriert, gründlich mit Eiswasser gewaschen und getrocknet (Sulfochlorid: 1. Fraktion). Das Filtrat wurde auf ein Viertel seines Volumens eingeengt und mit der fünffachen Menge Eiswasser versetzt. Das auskristallisierende Sulfochlorid wurde abfiltriert, mit Eiswasser gewaschen und getrocknet (2. Fraktion). Die beiden Sulfochlorid-Fraktionen wurden in 40 ml Dioxan gelöst und auf 40 °C erwärmt und bei dieser Temperatur mit einer Mischung aus 20 ml Ethanol und 20 ml konz. wässr. Ammoniak tropfenweise versetzt. Nach beendeter Zugabe wurde 1 Stunde bei 40 °C gerührt und danach 2 Stunden am Rückfluss gekocht. Nach dem Abkühlen wurde vom Teilprodukt (1. Fraktion) abfiltriert, dieses mit Wasser gewaschen und das Filtrat im Vakuum zur Trockne eingeengt. Der Rückstand wurde in Wasser suspendiert, abfiltriert und mit Wasser und mit wenig kaltem Ethanol gewaschen (2. Fraktion). Nach anschliessendem Trocknen wurden zusammen 10 g (70% d. Theorie) 3-Amino-4-methoxy-1,2-benzisothiazol-1,1-dioxid erhalten; Schmelzpunkt: 273–276 °C.

3-Amino-4-hydroxy-1,2-benzisothiazol-1,1-dioxid

5 g 3-Amino-4-methoxy-1,2-benzisothiazol-1,1-dioxid und 1,63 g Kaliumcyanid wurden in 15 ml Dimethylsulfoxid unter Rühren auf eine Temperatur von 130–140 °C (Badtemperatur 160 °C) erwärmt und 1,5 Stunden bei dieser Temperatur gehalten. Das abgekühlte Reaktionsgemisch wurde in 250 ml Methylenchlorid eingerührt und der ausgefallene Niederschlag wurde abfiltriert, getrocknet und in 30 ml Wasser gelöst. Die Lösung wurde bei 60 °C mit Aktivkohle behandelt und heiss filtriert. Das heisse Filtrat wurde mit konzentrierter wässriger Salzsäure auf pH 1 gebracht. Nach dem Abkühlen wurde das auskristallisierte Produkt abgesaugt, mit wenig Eiswasser gewaschen und im Vakuum getrocknet. Man erhielt 43 g (92% d. Theorie) 3-Amino-305 °C (Zersetzung).

Beispiel 2

4-Hydroxy-1,2-benzisothiazol-3(2H)-on-1,1-dioxid-natriumsalz

10 g 4-Hydroxy-1,2-benzisothiazol-3(2H)-on-1,1-dioxid wurden in Isopropanol bei einer Temperatur von 60 °C gelöst und bei dieser Temperatur mit einer stöchiometrischen Menge Natronlauge (2,0 g in 6 ml Wasser) versetzt. Nach dem Abkühlen wurde das Natriumsalz des 4-Hydroxy-1,2-benzisothiazol-3(2H)-on-1,1-dioxids abfiltriert, mit Isopropanol und Ether gewaschen und getrocknet. Man erhielt 10,8 g (96% d. Theorie); Schmelzpunkt: > 350 °C.

IR(KBr): 3320 (NH und OH assoz.), 1730 (CO), 1330 und 1140 $cm^{-1}$ ($SO_2$).

1H-NMR(DMSO-$d_6$): $\delta$ = 10,6 (s,1,OH, austauschbar mit $DC_3OD$); 7,55 (dd, 1H, J=4Hz, 6-H); 7,20 und 7,05 (zwei d, je 1H, J=4Hz, 5-H und 7-H)

$C_7H_4NNaO_4S \times H_2O$ (239,19)

| | | | | |
|---|---|---|---|---|
| Ber.: | C 35,15 | H 2,52 | N 5,85 | S 13,41 |
| Gef.: | 35,36 | 2,40 | 5,88 | 13,45 |

Beispiel 3

4,6-Dihydroxy-1,2-benzisothiazol-3(2H)-on-1,1-dioxid

In 25 ml auf 60 °C erwärmte Polyphosphorsäure wurden 1,5 g (4,7 mmol) 2-(N-tert.-Butyl)-sulfamoyl-4,6-dimethoxy-benzoesäure eingetragen und 1 Std. bei 80 °C gerührt. Das Reaktionsgemisch wurde auf Eis gegossen und der entstandene Niederschlag wurde abfiltriert. Das Filtrat wurde dreimal mit Essigester extrahiert und die organische Phase wurde getrocknet und eingeengt. Zusammen mit dem oben erhaltenen Niederschlag wurde der Rückstand aus Methanol umkristallisiert und ergab 0,74 g (65% der Theorie) 4,6-Dimethoxy-1,2- benzisothiazol- 3(2H)-on-1,1-dioxid; Schmelzpunkt: 258 °C;

$C_9H_9NO_5S$ (243,25)

| | | | | |
|---|---|---|---|---|
| Ber.: | C 44,44 | H 3,93 | N 5,76 | S 13,18 |
| Gef.: | 44,70 | 3,75 | 5,97 | 13,32 |

Das erhaltene 4,6-Dimethoxyderivat wurde in 25 ml Ethylenchlorid aufgenommen und mit 3,05 g (12,2 mmol) Bortribromid versetzt. Nach 3 Std. Rückflusskochen wurden weitere 3,05 g Bortribromid zugegeben und nochmals 3 Std. am Rückfluss erhitzt. Das Reaktionsgemisch wurde in Methanol eingetragen und mehrmals unter erneuter Methanolzugabe zur Trockne eingedampft. Der Rückstand wurde in wässriger Natriumbicarbonatlösung aufgenommen. Diese Lösung wurde mit Essigester gewaschen, mit Salzsäure sauergestellt und mit Essigester extrahiert. Der Essigesterextrakt wurde getrocknet und eingeengt. Der verbleibende Rückstand ergab aus Ethylenchlorid umkristallisiert 0,31 g (48% der Theorie) 4,6-Dihydroxy- 1,2-benzisothiazol- 3(2H)-on-1,1-dioxid; Schmelzpunkt: 286 °C;

IR(KBr): 3420, 3260 (OH, NH), 1700 (CO), 1330 und 1145 $cm^{-1}$ ($SO_2$).

1H-NMR($CDCl_3$ + $CD_3OD$): $\delta$ = 6,8(d, 1H, J=1,5Hz) und 6,57(d, je 1H, J=1,5Hz, 3-H und 5-H).

$C_7H_5NO_5S$ (215,19)

| | | | | |
|---|---|---|---|---|
| Ber.: | C 39,07 | H 2,34 | N 6,51 | S 14,90 |
| Gef.: | 39,15 | 2,60 | 6,58 | 14,95 |

Die Herstellung der Ausgangsverbindung 2- (N-tert.-Butyl)-sulfamoyl- 4,6-dimethoxy-1,2-benzoesäure erfolgte ausgehend vom 3,5-Dimethoxy-anilin nach dem folgenden Weg:

3,5-Dimethoxybenzolsulfonsäurechlorid

Zu einer Lösung von 26,6 g (0,174 mol) 3,5-Dimethoxyanilin 100 ml Eisessig gab man unter Kühlung (Reaktionstemperatur maximal +20 °C) 50 ml konz. wässr. Salzsäure. Unter Kühlung und bei einer Temperatur von −8 bis −3 °C tropfte man innerhalb von 35 Minuten hierzu eine Lösung von 12 g (0,174 mol) Natriumnitrit in 25 ml Wasser. Nachdem man eine Stunde im Kältebad nachgerührt hatte wurde filtriert und das dunkelbraun gefärbte Filtrat bei einer Temperatur von 20–25 °C in eine gerührte Suspension von 10,2 g (60 mmol) Kupfer(II)-chlorid und 2 g (20 mmol) Kupfer(I)-chlorid in 120 ml einer 30%igen Lösung von Schwefeldioxid in Eisessig gegossen. Die Reaktionstemperatur stieg zunächst auf 40 °C an und nach ihrem Absinken wurde noch 90 Min. auf 40 °C erwärmt. Anschliessend wurde das Reaktionsgemisch weitgehend eingeengt und auf 2 l Eiswasser gegossen. Man extrahierte dreimal mit Ether und wusch die organischen Extrakte mit Wasser, Natriumbicarbonat-Lösung und erneut mit Wasser.

Die Etherphase wurde nach dem Trocknen und Behandeln mit Aktivkohle eingeengt und ergab 19,8 g (48,5%) 3,5-Dimethoxybenzolsulfonsäurechlorid. Eine Probe wurde durch Umkristallisation aus Cyclohexan gereinigt; Schmelzpunkt: 74 °C.

N-tert.-Butyl-3,5-dimethoxy-benzolsulfonamid

Zu einer Lösung von 10,0 g (42 mmol) 3,5-Dimethoxy-benzolsulfonsäurechlorid in 100 ml Dioxan tropfte man eine Lösung von 7,3 g (0,1 mol) tert.-Butylamin in 20 ml Dioxan bei einer Temperatur von 40 °C innerhalb von 15 Minuten. Sobald die Reaktionstemperatur abzusinken begann, erwärmte man 1,5 Std. auf 45 °C und 2 Std. auf 60 °C. Nach dem Abkühlen wurde filtriert und das Filtrat im Vakuum eingedampft. Der Rückstand wurde aus Cyclohexan umkristallisiert und ergab 7,5 g (64% d. Theorie) N-tert.-Butyl-3,5-dimethoxy-benzolsulfonamid; Schmelzpunkt: 140 °C (aus Ethanol)

$C_{12}H_{19}NO_4S$ (273,36)

| | | | | |
|---|---|---|---|---|
| Ber.: | C 52,73 | H 7,01 | N 5,12 | S 11,73 |
| Gef.: | 53,00 | 6,93 | 5,05 | 11,70 |

2-(N-tert.-Butyl)-sulfamoyl-4,6-dimethoxy-benzoesäure

15,3 ml einer 15%igen Lösung von n-Butyllithium in Hexan (25 mmol) wurden innerhalb von 10 Min. bei einer Temperatur von −10 bis −3 °C zu einer Lösung von 2,5 g (25 mmol) frisch destilliertem Diisopropylamin in 40 ml wasserfreiem Tetrahydrofuran getropft. Man rührte 20 Min. nach, kühlte dann auf −70 °C und tropfte bei dieser Temperatur innerhalb von 40 Min. 1,7 g (6,2 mmol) N-tert.-Butyl-3,5-dimethoxy-benzolsulfonamid, gelöst in 50 ml Tetrahydrofuran, zu. Nach beendeter Zugabe liess man das Reaktionsgemisch auf Raumtemperatur erwärmen und rührte noch 1 Stunde.

Anschliessend kühlte man erneut auf −70 °C und leitete bei dieser Temperatur einen Kohlendioxid-Strom über das Reaktionsgemisch. Nach beendeter Reaktion (Wärmetönung) liess man unter weiterem Kohlendioxidstrom auf Raumtemperatur erwärmen. Das Reaktionsgemisch wurde im Vakuum eingeengt und der feste Rückstand wurde zwischen Wasser und Ether verteilt. Die organische Phase ergab nach Waschen mit Wasser und Einengen 1,1 g Ausgangsverbindung, die erneut in die Reaktion eingesetzt wurden. Die wässrige Phase wurde mit Salzsäure angesäuert. Der entstehende Niederschlag wurde aus Benzol umkristallisiert und ergab 0,5 g (86% Ausbeute, berechnet auf umgesetztes Ausgangsmaterial) 2-(N-tert.-Butyl)- sulfamoyl- 4,6-dimethoxy-benzoesäure; Schmelzpunkt: 209 °C (unter Zersetzung). IR(KBr): 1730, 1710 (CO), 1320 und 1140 $cm^{-1}$ ($SO_2$) 1H-NMR ($DMSO-d_6$+$CD_3OD$): $\delta$ = 7,15 (d, 1H, J=1,5Hz) und 6,90 (d, 1H, J=1,5Hz, 3-H und 5-H); 4,05 (s, 3H) und 4,08 (s, 3H; 4-$OCH_3$ und 6-$OCH_3$); 1,18 (s, 9H, $C(CH_3)_3$).

$C_{13}H_{19}NO_6S$ (317,37)

| | | | | |
|---|---|---|---|---|
| Ber.: | C 49,20 | H 6,04 | N 4,41 | S 10,10 |
| Gef.: | 48,96 | 6.07 | 4,35 | 9,95 |

Beispiel 4

4-Hydroxy-1,2-benzisothiazol-3(2H)-on-1,1-dioxid

2,5 g (11,8 mmol) 3-Amino-4-methoxy-1,2-benzisothiazol-1,1-dioxid wurden in einer Lösung von 0,47 g (11,8 mmol) Natriumhydroxid in 300 ml Wasser 3 Std. am Rückfluss erhitzt. Das Reaktionsgemisch wurde heiss filtriert und mit Salzsäure angesäuert. Der entstandene Niederschlag wurde abfiltriert, getrocknet und aus Benzol/Essigester umkristallisiert. Man erhielt 2,0 g (9,4 mmol) 4-Methoxy-1,2-benzisothiazol-3(2H)-on-1,1-dioxid (Schmelzpunkt: 224 °C), die in 500 ml Ethylenchlorid gelöst wurden und bei 0 °C mit 10 g (40 mmol) Bortribromid versetzt wurden. Das Reaktionsgemisch wurde langsam erwärmt und 1 Std. unter Rückfluss erhitzt. Anschliessend engte man im Vakuum ein und dampfte mehrmals unter Zugabe von Ethanol im Vakuum ein. Das erhaltene Produkt wurde aus Wasser umkristallisiert und ergab 1,6 g (68% d. Theorie) 4-Hydroxy-1,2-benzisothiazol-3(2H)-on-1,1-dioxid; Schmelzpunkt: 228 °C

$C_7H_5NO_4S$ (199,19)

| | | | | |
|---|---|---|---|---|
| Ber.: | C 42,20 | H 2,53 | N 7,03 | S 16,10 |
| Gef.: | 41,95 | 2,50 | 7,04 | 16,08 |

Beispiel 5

4-Hydroxy-1,2-benzisothiazol-3(2H)-on-1,1-dioxid

2,5 g (11,8 mmol) 3-Amino-4-methoxy-1,2-benzisothiazol-1,1-dioxid wurden analog Beispiel 4 mit Natriumhydroxid umgesetzt und durch anschliessendes Ansäuren in 4-Methoxy-1,2-benzisothiazol-3(2H)-on-1,1-dioxid überführt, das anschliessend mit 20 g Pyridin-Hydrochlorid auf 200–205 °C erhitzt wurde. Das Reaktionsgemisch wurde nach dem Erkalten mit Benzol mehrfach ausgekocht, in Wasser gelöst und die Lösung ebenfalls mit Benzol ausgekocht. Die vereinigten Benzolphasen wurden getrocknet und im Vakuum eingeengt. Der Rückstand wurde aus Wasser umkristallisiert und ergab 1,35 g (57,5% der Theorie) 4-Hydroxy-1,2-benzisothiazol-3(2H)-on-1,1-dioxid; Schmelzpunkt: 228 °C

$C_7H_5NO_4S$ (199,19)

| | | | | |
|---|---|---|---|---|
| Ber.: | C 42,20 | H 2,53 | N 7,03 | S 16,10 |
| Gef.: | 41,90 | 2,45 | 7,18 | 16,30 |

Beispiel 6

4-Hydroxy-1,2-benzisothiazol-3(2H)-on-1,1-dioxid

2,5 g (11,8 mmol) 3-Amino-4-methoxy-1,2-benzisothiazol-1,1-dioxid wurden analog Beispiel 5 mit Natriumhydroxid umgesetzt und durch anschliessendes Ansäuren in 4-Methoxy-1,2-benzisothiazol-3(2H)-on-1,1-dioxid überführt. Anschliessend wurde diese Verbindung zusammen mit 650 mg (10 mmol) Kaliumcyanid in Dimethylsulfoxid 3 Std. auf 180 °C erwärmt. Durch Zugabe von Petrolether wurde das Kaliumsalz des Produkts ausgefällt, und dieses wurde in Wasser gelöst. Durch Ansäuern mit wässr. Salzsäure, Abfiltrieren und Umkristallisieren erhielt man 1,46 g (62% der Theorie) 4-Hydroxy-1,2-benzisothiazol-3(2H)-on-1,1-dioxid; Schmelzpunkt: 228 °C (aus Wasser).

$C_7H_5NO_4S$ (199,19)

| | | | | |
|---|---|---|---|---|
| Ber.: | C 42,20 | H 2,53 | N 7,03 | S 16,10 |
| Gef.: | 42,18 | 2,58 | 7,06 | 16,18 |

Beispiel 7

4-Hydroxy-1,2-benzisothiazol-3(2H)-on-1,1-dioxid

2,5 g (11,8 mmol) 3-Amino-4-methoxy-1,2-benzisothiazol-1,1-dioxid wurden analog Beispiel 6 umgesetzt. Anstelle von Kaliumcyanid wurde Kaliumbromid verwendet. Man erhielt 1,05 g (45% der Theorie) 4-Hydroxy-1,2-benzisothiazol-3(2H)-on-1,1-dioxid; Schmelzpunkt: 228 °C (aus Wasser).

$C_7H_5NO_4S$ (199,19)

| | | | | |
|---|---|---|---|---|
| Ber.: | C 42,20 | H 2,53 | N 7,03 | S 16,10 |
| Gef.: | 42,00 | 2,61 | 7,09 | 16,21 |

Beispiel 8

4-Hydroxy-1,2-benzisothiazol-3(2H)-on-1,1-dioxid

8,1 g (40 mmol) 3-Methoxy-2-methyl-benzolsulfonamid und 5,5 g (52 mmol) wasserfreies Soda wurden mit 300 ml Wasser versetzt und anschliessend wurde eine Lösung von 15,9 g (500 mmol) Kaliumpermanganat in 400 ml Wasser bei einer Temperatur von 70 bis 80 °C tropfenweise zugesetzt. Danach wurde das Reaktionsgemisch bis zur Entfärbung (ca. 1–2 Std). am Rückfluss erhitzt und noch heiss filtriert. Das Filtrat wurde im Vakuum auf das halbe Volumen eingeengt und mit konz. wässr. Salzsäure angesäuert. Zuerst kristallisierte eine kleine Menge Ausgangssubstanz aus und nach Stehen über Nacht konnte von auskristallisiertem Produkt abfiltriert werden.

Durch Extraktion der wässrigen Mutterlauge mit Ether konnte man eine weitere Menge an Produkt isolieren. Nach Umkristallisation aus Benzol/Essigester erhielt man 4,8 g (56% d. Theorie) 4-Methoxy-1,2-benzisothiazol-3(2H)-on-1,1-dioxid (Schmelzpunkt: 225 °C), die in 1000 ml Ethylenchlorid gelöst wurden und bei −10 °C mit 11,3 g (45 mmol) Bortribromid versetzt wurden. Das Reaktionsgemisch wurde danach 30 min. bei Raumtemperatur gerührt und 1 Std. unter Rückfluss erwärmt. Anschliessend engte man im Vakuum ein, erhitzte den Rückstand mit 150 ml 70%igem Ethanol 15 min. und engte im Vakuum mehrmals unter Zugabe von Ethanol ein. Man erhielt 3,4 g (75% d. Theorie) 4-Hydroxy-1,2-benzisothiazol-3(2H)-on-1,1-dioxid; Schmelzpunkt: 228 °C (aus Wasser).

$C_7H_5NO_4S$ (199,19)

| | | | | |
|---|---|---|---|---|
| Ber.: | C 42,20 | H 2,53 | N 7,03 | S 16,10 |
| Gef.: | 42,10 | 2,59 | 7,06 | 16,20 |

Die Ausgangsverbindung 4-Methoxy-1,2-benzisothiazol-3(2H)-on-1,1-dioxid erhielt man aus 2-Methoxy-6-nitro-toluol auf folgende Weise:

3-Methoxy-2-methyl-anilin

200 g (1,19 mol) 2-Methoxy-6-nitro-toluol wurden in 2,5 l Ethanol in Gegenwart von 25 g Raney-Nickel bei 100 bar bis zur Aufnahme der theoretischen Wasserstoffmenge hydriert. Das Reaktionsgemisch wurde filtriert und im Vakuum eingeengt.

Die anschliessende Destillation lieferte 158,3 g (97% d. Theorie) 3-Methoxy-2-methylanilin; Siedepunkt: 126 °C bei 16,25 mbar.

3-Methoxy-2-methyl-benzolsulfonamid

10,0 g (73 mmol) 3-Methoxy-2-methyl-anilin wurden in 50 ml mit gasförmigem Chlorwasserstoff gesättigter, 50%ig wässriger Essigsäure gelöst und mit 5,5 g (80 mmol) Natriumnitrit (gelöst in 10 ml Wasser) diazotiert. Ein geringer Nitritüberschuss wurde mit Harnstoff beseitigt. Die Diazoniumsalzlösung wurde noch mit 6 g Magnesiumsulfat versetzt und anschliessend auf 60 ml mit Schwefeldioxid gesättigten Eisessig gegossen, dem zuvor 3 g Kupfer(I)-chlorid zugesetzt worden waren.

Das Reaktionsgemisch wurde langsam auf Zimmertemperatur erwärmt und 4 Std. gerührt. Nach Zusatz von 500 ml Wasser wurde das Rohprodukt mit Methylenchlorid extrahiert. Die organischen Phasen wurden mit Wasser, mit Natriumbicarbonatlösung und erneut mit Wasser gewaschen, getrocknet, eingeengt und der Rückstand im Vakuum destilliert. Das bei einem Druck von 19,5 mbar und bei einem Siedepunkt von 110 bis 135 °C erhaltene Destillat (Sulfonsäurechlorid) wurde in 50 ml Ethanol aufgenommen und mit konz. wässr. Ammoniak versetzt. Das Reaktionsgemisch wurde 15 Min. gerührt und im Vakuum eingeengt. Der Rückstand wurde aus Methanol/Wasser umkristallisiert und man erhielt 4,6 g (31% d. Theorie) 3-Methoxy-2-methyl-benzolsulfonamid; Schmelzpunkt: 193 °C.

Beispiel 9

4-Hydroxy-1,2-benzisothiazol-3(2H)-on-1,1-dioxid

6,6 g (30 mmol) 4-Chlor-1,2-benzisothiazol-3(2H)-on-1,1-dioxid, 12,3 g (90 mmol) Kaliumcarbonat, 0,6 g Kupferpulver und 1,5 g Kupfer(II)-chlorid wurden in 110 ml Wasser 12 Stunden in einem Glasautoklaven auf 150 °C erhitzt. Danach wurde das Reaktionsgemisch mit Wasser verdünnt, erwärmt und heiss filtriert. Das Filtrat wurde mit Salzsäure angesäuert und mit Ether extrahiert. Die Etherextrakte wurden eingeengt und der erhaltene Rückstand aus Wasser umkristallisiert. Man erhielt 4,3 g (72% d. Theorie) 4-Hydroxy-1,2-benzisothiazol-3 (2H)-on-1,1 -dioxid; Schmelzpunkt: 228 °C.

$C_7H_5NO_4S$ (199,19)

| | | | | |
|---|---|---|---|---|
| Ber.: | C 42,20 | H 2,53 | N 7,03 | S 16,10 |
| Gef.: | 42,10 | 2,59 | 7,06 | 16,20 |

Die Herstellung der Ausgangsverbindung 4-Chlor-1,2-benzisothiazol-3(2H)-on-1,1-dioxid erfolgt ausgehend vom 2-Chlor-6-nitro-toluol nach einem der folgenden Verfahren:

WEG A:

3-Chlor-2-methyl-anilin

100 g (0,583 mol) 2-Chlor-6-nitro-toluol wurden in einer Suspension von 10 g Raney-Nickel in 1 l Ethanol hydriert. Nachdem die berechnete Menge Wasserstoff aufgenommen wurde, wurde das Reaktionsgemisch filtriert und das Filtrat im Vakuum eingedampft.

Der Rückstand wurde bei einem Vakuum von 14 mbar destilliert. Die bei 135–140 °C überdestillierende Substanz ist das 3-Chlor-2-methyl-anilin; Ausbeute 72 g (87% d. Theorie); Siedepunkt 140 °C/14,3 mbar.

3-Chlor-2-methyl-benzolsulfonsäurechlorid

30 g (0,212 mol) 3-Chlor-2-methyl-anilin wurden mit 50 ml konz. wässr. Salzsäure versetzt. Man kühlte auf −7 bis −3 °C und tropfte bei dieser Temperatur innerhalb von 1 Std. eine Lösung von 14,6 g (0,212 mol) Natriumnitrit in 50 ml Wasser zu. Man rührte eine weitere Stunde bei 0 °C, versetzte danach mit 20,3 g (0,1 mol) Magnesiumchlorid-Hexahydrat, rührte 10 Minuten und filtrierte das Reaktionsgemisch. Das Filtrat wurde in einem dünnen Strahl zu einer Suspension von 12 g (71 mmol) Kupfer(II)-chlorid in 250 ml einer 30%igen Lösung von Schwefeldioxid in Eisessig gegossen. Das Reaktionsgemisch erwärmte sich während dieser Zugabe von 20 °C auf 40 °C unter einer starken Gasentwicklung. Man rührte noch 60 Min. bei Raumtemperatur und 60 Min. bei 35 °C. Anschliessend wurde mit 500 g Eis versetzt, bis zum Auftauen des Eises gerührt und filtriert. Man erhielt 38,2 g (80% der Theorie) 3-Chlor-2-methylbenzolsulfonsäurechlorid, das direkt in die nachfolgende Reaktion eingesetzt werden konnte.

3-Chlor-2-methyl-benzolsulfonamid

38,2 g (0,17 mol) 3-Chlor-2-methyl-benzolsulfonsäurechlorid wurden in 200 ml Dioxan gelöst und auf 40 °C erwärmt. Innerhalb von 15 Minuten tropfte man eine Mischung aus 100 ml konz. wässr. Ammoniak und 100 ml Ethanol hinzu, wobei die Temperatur des Reaktionsgemisches auf 55 °C anstieg.

Anschliessend wurde noch 2 Std. auf 70 °C erwärmt und danach abgekühlt und filtriert. (Der Rückstand bestand aus 3,3′-Dichlor-2,2′-dimethyldiphenyldisulfid). Das Filtrat wurde im Vakuum eingeengt und mit Eiswasser versetzt. Der Niederschlag wurde abgesaugt und mit Eiswasser versetzt. Der Niederschlag wurde abgesaugt und mit Eiswasser gewaschen und ergab nach Umkristallisation aus Ethanol/Wasser 24 g (69% d. Theorie) 3-Chlor-2-methyl-benzolsulfonamid;

Schmelzpunkt: 179 °C.

$C_7H_8ClNO_2S$ (205,67)

| | | | | | |
|---|---|---|---|---|---|
| Ber.: | C 40,88 | H 3,92 | Cl 17,24 | N 6,81 | S 15,59 |
| Gef.: | 40,90 | 3,85 | 17,33 | 6,71 | 15,63 |

Durch Ansäuern mit konz. wässr. Salzsäure erhielt man aus dem Filtrat 3-Chlor-2-methyl-benzolsulfinsäure, Schmelzpunkt: 112 °C, Ausbeute 7,0 g entsprechend 22% d. Theorie. Diese Sulfinsäure liess sich mit Kupfer(II)-chlorid in Ameisensäure mit 88%iger Ausbeute in das 3-Chlor-2-methylbenzolsulfonsäurechlorid überführen (40 °C, 1 Stunde, Aufarbeitung durch Versetzen mit konz. wässr. Salzsäure und Eis) und somit ebenfalls in das 3-Chlor-2-methyl-benzolsulfonamid. Die Gesamtausbeute stieg dann auf 85% d. Theorie.

4-Chlor-1,2-benzisothiazol-3(2H)-on-1,1-dioxid

16,4 g (80 mmol) 3-chlor-2-methyl-benzolsulfonamid wurden zusammen mit 14 g (100 mmol) Kaliumcarbonat und 33,2 g (210 mmol) Kaliumpermanganat in 350 ml Wasser 1 Stunde am Rückfluss erhitzt. Das Reaktionsgemisch wurde heiss filtriert und der Filterrückstand mit heissem Wasser gewaschen. Die vereinigten Filtrate wurden mit konz. wässr. Salzsäure angesäuert und gekühlt. Das Kristallisat wurde abgesaugt, mit Wasser gewaschen und getrocknet und ergab 12,6 g (72,5% d. Theorie) 4-Chlor-1,2-benzisothiazol-3(2H)-on-1,1-dioxid; Schmelzpunkt: 210 °C (aus Wasser).

IR(KBr): 3420 (NH), 1730 (CO), 1330 und 1180 $cm^{-1}$ ($SO_2$) $C_7H_4ClNO_3S$ (217,64)

| | | | | | |
|---|---|---|---|---|---|
| Ber.: | C 38,63 | H 1,85 | Cl 16,29 | N 6,44 | S 14,74 |
| Gef.: | 38,70 | 1,80 | 15,90 | 6,70 | 14,85 |

WEG B:

2-Chlor-6-nitro-benzoesäure

Ein Gemisch aus 77,7 g (0,453 mol) 2-Chlor-6-nitro-toluol, 190 g (1,2 mol) Kaliumpermanganat, 500 ml 1 N Kalilauge und 4 l Wasser wurde 8,5 Std. auf 100 °C erhitzt. Nach Stehen über Nacht wurden 25 g (0,16 mol) Kaliumpermanganat zugesetzt und das Reaktionsgemisch weitere 2,5 Std. auf 100 °C erhitzt. Danach wurde mit Wasserdampf das nicht umgesetzte Ausgangsmaterial abdestilliert. Die verbleibende Reaktionslösung wurde filtriert, der Filterrückstand mit heissem Wasser gewaschen und die Filtrate auf ca. 750 ml eingeengt. Nach dem Ansäuern mit Salzsäure erhielt man einen Niederschlag, der abfiltriert und getrocknet wurde. Man erhielt 45,9 g (50% d. Theorie) 2-Chlor-6-nitro-benzoesäure; Schmelzpunkt: 166 °C (aus Ethylenchlorid).

2-Chlor-6-nitro-benzoesäurechlorid

Zu einer Suspension von 20,1 g (0,1 mol) 2-Chlor-6-nitrobenzoesäure in 150 ml wasserfreiem Benzol gab man 1 ml Dimethylformamid und 17,9 g (0,15 mol) Thionylchlorid. Nach 30 Min. Rühren erwärmte man auf Rückflusstemperatur und gab nach 3 Std. weitere 17,9 g (0,15 mol) Thionylchlorid zu. Nach weiteren 3 Std. Rückflusskochen wurde nach dem Erkalten filtriert und das Filtrat im Vakuum eingeengt. Man erhielt 18,9 g (85,9% d. Theorie) 2-Chlor-6-nitro-benzoesäurechlorid als Öl.

2-Chlor-6-nitro-benzoesäuremethylester

Innerhalb von 10 Min. wurden 18,9 g (86 mmol) 2-Chlor-6-nitro-benzosäurechlorid in 100 ml Methanol eingetropft. Nach 4 Std. Rückflusserhitzen wurde im Vakuum eingeengt. Der Rückstand wurde mit wenig eiskaltem Methanol behandelt und abfiltriert. Man erhielt 12,3 g (66% d. Theorie) 2-Chlor-6-nitro-benzoesäuremethylester; Schmelzpunkt: 98 °C (aus Cyclohexan).

6-Chlor-anthranilsäuremethylester

4,5 g (0,212 mol) 6-Chlor-2-nitro-benzoesäuremethylester wurden in 1,8 l Methanol mit Raney-Nickel bis zur berechneten Wasserstoffaufnahme hydriert. Das filtrierte Reaktionsgemisch wurde im Vakuum eingeengt und der Rückstand im Vakuum destilliert. Man erhielt 34,6 g (88% d. Theorie) 6-Chloranthranilsäuremethylester; Siedepunkt 158 °C bei 14,3 mbar.

6-Chlor-2-chlorsulfonyl-benzoesäuremethylester

34,6 g (0,186 mol) 6-Chloranthranilsäuremethylester wurden unter Kühlung in 70 ml konz. wässr. Salzsäure unter Rühren eingetragen und bei einer Temperatur von 0 bis +10 °C mit einer Lösung von 12,8 g (0,186 mol) Natriumnitrit in 30 ml Wasser diazotiert. Man rührte das Reaktionsgemisch 45 Min. bei 0 °C und goss es anschliessend in eine gerührte Suspension von 2 g Kupfer(I)-chlorid und 17 g Kupfer(II)-chlorid in 160 ml einer 30%igen Lösung von Schwefeldioxid in Eisessig. Nach beendeter Stickstoffentwicklung wurde noch 2 Std. bei Raumtemperatur gerührt. Es wurde mit Ether extrahiert und die organischen Extrakte gewaschen, getrocknet und eingeengt. Man erhielt 38,2 g (76% d. Theorie) 6-Chlor-2-chlorsulfonyl-benzoesäuremethylester.

4-Chlor-1,2-benzisothiazol-3(2H)-on-1,1-dioxid

Zu einer Lösung von 38,2 g (0,141 mol) 6-Chlor-2-chlorsulfonylbenzoesäuremethylester in 150 ml Dioxan wurden innerhalb von 20 Min. bei einer Temperatur von 30 bis 50 °C 150 ml mit Ammoniak gesättigtes Ethanol getropft. Anschliessend wurde 2 Std. auf 40 °C und 2 weitere Std. auf 60 °C erwärmt. Nach dem Erkalten wurde eingeengt, mit Ether behandelt und filtriert. Der Filterrückstand wurde mit Wasser gekocht. Es wurde heiss filtriert und das Filtrat mit Salzsäure angesäuert. Man erhielt 19,8 g (64,5% der Theorie) 4-Chlor-1,2-benzisothiazol-3(2H)-on-1,1-dioxid; Schmelzpunkt: 210 °C.

$C_7H_4ClNO_3S$ (217,64)

| | | | | | |
|---|---|---|---|---|---|
| Ber.: | C 38,63 | H 1,85 | Cl 16,29 | N 6,44 | S 14,73 |
| Gef.: | 38,65 | 1,98 | 16,20 | 6.41 | 14,75 |

Beispiel 10

4-Hydroxy-1,2-benzisothiazol-3(2H)-on-1,1-dioxid

In 50 g auf 60 °C erwärmte Polyphosphorsäure wurden innerhalb von 5 Min. 3,4 g (12 mmol) 2-(N-tert.-Butyl)-sulfamoyl-6-methoxybenzoesäure eingetragen. Anschliessend wurde noch 20 Min. auf 70 °C erwärmt und in die noch warme Reaktionsmischung Eis gegeben. Der entstandene Niederschlag wurde abfiltriert und aus Wasser umkristallisiert (1,6 g; Schmelzpunkt: 224 °C; $C_8H_7NO_4S$: 4-Methoxy- 1,2-benzisothiazol-3(2H)-on 1,1-dioxid).

Das Kristallisat wurde mit 24 ml (25 mmol) Bortribromid in 400 ml Ethylenchlorid eine Std. bei Raumtemperatur und 2 Std. bei Rückflusstemperatur umgesetzt. Anschliessend wurde das Reaktionsgemisch eingedampft und der Rückstand mehrmals mit Methanol versetzt und abgedampft. Der Rückstand wurde aus Isopropanol umkristallisiert und ergab 1,4 g (58% d. Theorie) 4-Hydroxy-1,2-benzisothiazol-3(2H)-on-1,1-dioxid; Schmelzpunkt: 224 °C.

$C_7H_5NO_4S$ (199,19)

| | | | | |
|---|---|---|---|---|
| Ber.: | C 42,20 | H 2,53 | N 7,09 | S 16,10 |
| Gef.: | 42,16 | 2,57 | 6,99 | 16,01 |

Die Herstellung der Ausgangsverbindung 2-(N-tert.-Butyl)-sulfamoyl-6-methoxy-benzoesäure erfolgte auf dem folgenden Wege ausgehend von m-Anisidin:

3-Methoxy-benzolsulfonsäurechlorid

61,6 g (0,5 mol) m-Anisidin wurden in 50 ml Eisessig gelöst und tropfenweise mit 110 ml konz. wässr. Salzsäure versetzt. Man kühlte das Gemisch auf 0 °C und diazotierte mit einer Lösung von 38 g (0,551 mol) Natriumnitrit in 75 ml Wasser. Dieses Gemisch rührte man noch 10–15 Min. und goss es langsam in eine Mischung aus 24 g (0,14 mol) Kupfer(II)-chlorid und 5 g Kupfer(I)-chlorid in 300 ml einer 30%igen Lösung von Schwefeldioxid in Eisessig und 450 ml Benzol, wobei eine starke Gasentwicklung und ein Temperaturanstieg auf 40 °C beobachtet wurde. Sobald die Reaktionstemperatur abfiel wurde noch 1 Stunde auf 40 °C erwärmt. Nach dem Abkühlen wurde mit 2,5 l Eiswasser verdünnt und nach Filtrieren die organische Phase abgetrennt. Diese wurde mit Wasser und Natriumbicarbonatlösung gewaschen und eingeengt. Man erhielt 84,5 g (82% der Theorie) 3-Methoxy-benzolsulfonsäurechlorid.

N-tert.-Butyl-3-methoxy-benzolsulfonamid

49,5 g (0,24 mol) 3-Methoxy-benzolsulfonsäurechlorid wurden in 50 ml Tetrahydrofuran gelöst und innerhalb von 25 Min. zu einer Lösung 40 g (0,55 mol) tert.-Butylamin in 200 ml Tetrahydrofuran getropft. Es wurde 1 Std. bei Raumtemperatur und 7 Std. bei 60 °C gerührt. Das Reaktionsgemisch wurde filtriert und das Filtrat im Vakuum eingeengt. Der Rückstand wurde mit Cyclohexan behandelt und ergab 54,1 g (92,6% der Theorie) N-tert.-Butyl-3-methoxy-benzolsulfonamid; Schmelzpunkt: 100 °C.

2-(N-tert.-Butyl)-sulfamoyl-6-methoxy-benzoesäure

Eine Lösung von 3,3 g (14 mmol) N-tert.-Butyl-3-methoxybenzolsulfonamid in 70 ml wasserfreiem Tetrahydrofuran wurde unter Stickstoff bei einer Temperatur von −60 bis −50 °C innerhalb von 15 Min. tropfenweise mit 19 ml einer 15%igen Lösung von n-Butyllithium in Hexan (31 mmol) versetzt. Anschliessend wurde das Reaktionsgemisch 1 Std. bei 0 °C gerührt und nach erneutem Kühlen auf −70 bis −50 °C wurde ein Kohlenstoffdioxidstrom übergeleitet. Die Kohlenstoffdioxidbehandlung wurde nach beendeter exothermer Reaktion noch 1 Std. bei Raumtemperatur fortgeführt. Danach wurde das Reaktionsgemisch im Vakuum eingedampft und der Rückstand zwischen Eiswasser und Ether verteilt. Die wässrige Schicht wurde mit Ether gewaschen und mit Salzsäure angesäuert. Der Niederschlag wurde abfiltriert und ergab 3,5 g (87% d. Theorie) 2-(N-tert.-Butyl)-sulfamoyl-6-methoxy-benzoesäure.

Beispiel 11

4-Hydroxy-1,2-benzisothiazol-3(2H)-on-1,1-dioxid

Eine Lösung von 1,2-Benzisothiazol-3(2H)-on-1,1-dioxid-4-diazoniumchlorid, die durch Diazotierung von 1,98 g (10 mmol) 4-Amino-1,2-benzisothiazol-3(2H)-on-1,1-dioxid mit Natriumnitrit in verdünnter Salzsäure hergestellt wurde, wurde mit 150 ml 10% wässriger Schwefelsäure versetzt und auf dem Wasserbad bis zur Beendigung der Stickstoffentwicklung erhitzt. Nach dem Abkühlen wurde noch mit konz. wässriger Salzsäure stark angesäuert und mit Ether extrahiert. Die organische Phase wurde eingeengt, der Rückstand in wenig verdünnter Natronlauge gelöst und nach Filtration wurde erneut mit Salzsäure angesäuert. Die sich abscheidenden Kristalle wurden aus Wasser umkristallisiert und ergaben 98 g (40% der Theorie) 4-Hydroxy-1,2-benzisothiazol-3(2H)-on-1,1-dioxid; Schmelzpunkt: 228 °C.

$C_7H_5NO_4S$ (199,19)

| | | | | |
|---|---|---|---|---|
| Ber.: | C 42,20 | H 2,53 | N 7,03 | S 16,10 |
| Gef.: | 42,14 | 2,57 | 7,09 | 15,98 |

Die Ausgangsverbindung 1,2-Benzisothiazol-3(2H)-on-1,1-dioxid wurde auf dem folgenden Wege hergestellt:

2-Methyl-3-nitro-benzolsulfonamid

7,5 g (49 mmol) 2-Amino-6-nitro-toluol wurden in einer Mischung aus 27,5 g konz. Schwefelsäure und 250 ml Wasser gelöst und mit einer Lösung von 3,75 g (54 mmol) Natriumnitrit in 7,5 ml Wasser bei einer Temperatur von 0 bis 5 °C diazotiert. Nach 2 Stunden wurde das Reaktionsgemisch in eine vorbereitete Mischung aus 90 ml einer 30%igen Schwefeldioxidlösung in Eisessig und einer Lösung von 1,5 g Kupfer(II)-chlorid in 4 ml Wasser portionsweise eingetragen. Die Temperatur während des Eintragens betrug 20 °C und die weitere Behandlung und Umsetzung mit Ammoniak erfolgte analog der Beschreibung des 3-Methoxy-2-methyl-benzolsulfonamids (Beispiel 8). Man erhält 8,2 g (77% der Theorie) 2-Methyl-3-nitro-benzolsulfonamid.

$C_7H_8N_2O_4S$ (216,21)

| | | | | |
|---|---|---|---|---|
| Ber.: | C 38,89 | H 3,73 | N 12,95 | S 14,83 |
| Gef.: | 38,67 | 3,79 | 12,72 | 14,81 |

4-Nitro-1,2-benzisothiazol-3(2H)-on-1,1-dioxid

8,0 g (37 mmol) 2-Methyl-3-nitro-benzolsulfonamid wurden mit Kaliumpermanganat in Wasser analog dem Beispiel 8 oxidiert. Nach der Etherextraktion erhält man durch Einengen und Umkristallisation aus Wasser 3,8 g (45% der Theorie) der Nitro-Verbindung; Schmelzpunkt: 239 °C.

4-Amino-1,2-benzisothiazol-3(2H)-on-1,1-dioxid

3,0 g (13 mmol) 4-Nitro-1,2-benzisothiazol-3(2H)-on-1,1-dioxid wurden in 100 ml Ethanol gelöst und über Wasserstoff unter Verwendung eines Palladium/Tierkohle-Katalysators hydriert. Nach Filtration, Einengen und Umkristallisation aus Ethanol wurden 2,3 g (89,5% der Theorie) der Aminoverbindung erhalten; Schmelzpunkt: 244–245 °C.

$C_7H_6N_2O_3S$ (198,19)

| | | | | |
|---|---|---|---|---|
| Ber.: | C 42,42 | H 3,05 | N 14,13 | S 16,18 |
| Gef.: | 42,31 | 3,12 | 14,01 | 16,18 |

1,2-Benzisothiazol-3(2H)-on-1,1-dioxid-4-diazoniumchlorid

1,98 g (10 mmol) 4-Amino-1,2-benzisothiazol-3(2H)-on-1,1-dioxid wurden unter Zusatz von Soda in Wasser gelöst und mit konz. wässriger Salzsäure gefällt. Zu dieser Suspension wurde bei 0 bis 5 °C eine Lösung von 700 mg Natriumnitrit in 3 ml Wasser zugetropft und 2 Stunden bei 5 °C gerührt. Die auf diese Weise erhaltene Diazoniumsalzlösung wurde für die weiteren Reaktionen ohne Isolierung des Diazoniumsalzes direkt eingesetzt.

Die Verbindungen der Formel I oder ihre Salze können in an sich üblicher Weise in Zubereitungsformen wie Tabletten, Pulver oder Lösungen eingearbeitet werden.

Die folgenden Beispiele mögen dies verdeutlichen:

Beispiel I

Süssstofflösung enthaltend 50 mg Natriumsalz des 4-Hydroxy-1,2-benzisothiazol-3(2H)-on-1,1-dioxids in 1 ml Lösung

In 100 ml destilliertem Wasser werden bei 60 °C nacheinander die folgenden Substanzen unter Rühren gelöst.:

0,1 g Sorbinsäure,
1,2 g Citronensäure,
1,5 g Dinatriumphosphat,
5,0 g Natriumsalz des Süssstoffes.

1 ml (entspricht ca. 25 Tropfen) enthalten 50 mg des obengenannten Süssstoffes, dies entspricht der Süsskraft von ca. 3 Stück Würfelzucker.

Beispiel II

Tabletten zum Süssen enthaltend 20 mg 4-Hydroxy-1,2-benzisothiazol-3(2H)-on-1,1-dioxid

Zusammensetzung einer Tablette:

| | |
|---|---|
| Süssstoff | 20 mg |
| Natriumhydrogencarbonat | 5 mg |
| Sorbit pulv. | 25 mg |

Der Süssstoff wird mit dem Natriumhydrogencarbonat und Sorbit gut vermischt und bei einer relativen Luftfeuchte von maximal 60% zu Tabletten verpresst.

1 Tablette entspricht der Süsskraft von etwa einem Stück Würfelzucker.

Beispiel III

Tabletten zum Süssen enthaltend 20 mg Natriumsalz des 4-Hydroxy-1,2-benzisothiazol-3(2H)-on-1,1-dioxids

Zusammensetzung einer Tablette:

| | |
|---|---|
| Natriumsalz des Süssstoffes | 20 mg |
| Sorbit | 30 mg |

Der Süssstoff wird mit dem Sorbit gut vermischt und zu Tabletten verpresst. 1 Tablette entspricht der Süsskraft von etwa einem Stück Würfelzucker.

**Patentansprüche für die Vertragsstaaten: BE, CH, DE, FR, GB, IT, LI, LU, NL, SE**

1. 4-Hydroxy-1,2-benzisothiazol-3(2H)-on-1,1-dioxide der allgemeinen Formel I

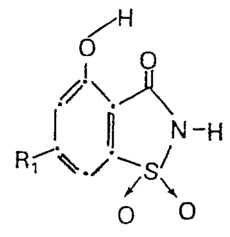

(I)

in der $R_1$ ein Wasserstoffatom oder die Hydroxygruppe bedeutet, und deren physiologisch unbedenkliche Alkali- oder Erdalkalisalze.

2. 4-Hydroxy-1,2-benzisothiazol-3(2H)-on-1,1-dioxid und dessen physiologisch unbedenkliche Alkali- oder Erdalkalisalze.

3. 4,6-Dihydroxy-1,2-benzisothiazol-3(2H)-on-1,1-dioxid und dessen physiologisch unbedenkliche Alkali- oder Erdalkalisalze.

4. Süssstoffzubereitungen, enthaltend eine oder mehrere Verbindungen der allgemeinen Formel I gemäss Anspruch 1 neben den üblichen Träger- und/oder Hilfsstoffen.

5. Süssstoffzubereitungen, enthaltend das Natrium- oder Calciumsalz des 4-Hydroxy-1,2-benzisothiazol-3(2H)-on-1,1-dioxids.

6. Süssstoffzubereitungen, enthaltend das Natrium- oder Calciumsalz des 4,6-Dihydroxy-1,2-benzisothiazol-3(2H)-on-1,1-dioxids.

7. Verwendung der Verbindungen der allgemeinen Formel I und/oder ihrer physiologisch verträglichen Salze gemäss Anspruch 1 zur Herstellung von Süssstoffzubereitungen.

8. Verfahren zur Herstellung von 4-Hydroxy-1,2-benzisothiazol-3(2H)-on-1,1-dioxiden der allgemeinen Formel I gemäss Anspruch 1, dadurch gekennzeichnet, dass

a) ein 3-Amino-1,2-benzisothiazol-1,1-dioxid der allgemeinen Formel II

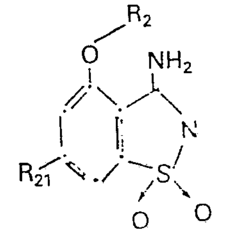

(II)

in der $R_2$ ein Wasserstoffatom, eine Alkyl- oder Aralkylgruppe und $R_{21}$ ein Wasserstoffatom, eine Hydroxy-, Alkoxy- oder Aralkoxygruppe bedeuten, in einer wässerigen Lösung mit Basen bei Temperaturen zwischen 50 °C und dem Siedepunkt der Lösung verseift wird und, falls $R_2$ eine Alkyl- oder Aralkylgruppe und/oder $R_{21}$ eine Alkoxy- oder Aralkoxygruppe bedeuten, das so erhaltene Produkt anschliessend einer Etherspaltung unterworfen wird oder

b) ein o-Sulfamoyl-o'-alkoxy(oder o'-aralkoxy)-toluol der allgemeinen Formel III

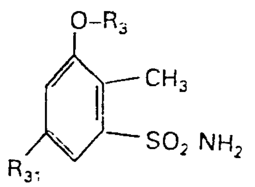

(III)

in der $R_3$ eine Alkyl- oder Aralkylgruppe und $R_{31}$ ein Wasserstoffatom oder eine Alkoxy- oder Aralkoxygruppe bedeuten, mit einem üblichen Oxidationsmittel oxidiert und das so erhaltene Produkt anschliessend einer Etherspaltung unterworfen wird, oder

c) in einem 1,2-Benzisothiazol-3(2H)-on-1,1-dioxid der allgemeinen Formel IV

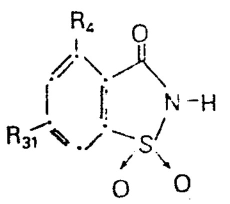

(IV)

in der $R_{31}$ wie oben definiert ist, und $R_4$ eine nucleophil austauschbare Gruppe bedeutet, der Rest $R_4$ gegen die Hydroxygruppe nucleophil ausgetauscht wird, wobei der Austausch eines Diazoniumkations in einer sauren wässerigen Lösung bei der Siedetemperatur der Lösung und der Austausch anderer austauschbarer Gruppen mit Alkalibasen in Gegenwart von Katalysatoren und in Anwesenheit von Wasser bei Temperaturen zwischen 120 und 200 °C erfolgt, und, falls $R_{31}$ eine Alkoxy- oder Aralkoxygruppe darstellt, das so erhaltene Produkt anschliessend einer Ätherspaltung unterworfen wird, oder

d) ein 2-Sulfamoyl-3-carbonsäurederivat der allgemeinen Formel V

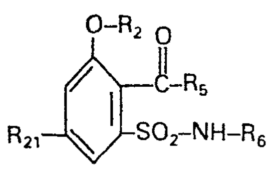

(V)

in der $R_{21}$ ein Wasserstoffatom, eine Hydroxy-, Alkoxy- oder Aralkoxygruppe und $R_2$ ein Wasserstoffatom oder eine Alkyl- oder Aralkylgruppe bedeutet, $R_6$ ein Wasserstoffatom oder einen tert.-Alkylrest mit 4 bis 19 Kohlenstoffatomen und $R_5$ eine Hydroxygruppe, eine Alkoxygruppe mit 1 bis 10 Kohlenstoffatomen, eine Aryloxygruppe, worin Aryl für Phenyl oder Naphthyl steht, eine Phenyl- oder Naphthylalkoxygruppe mit 1 bis 3 Kohlenstoffatomen im Alkylenteil oder ein Halogenatom oder eine andere, als nucleophil austauschbar vorbekannte Gruppe bedeuten, in Gegenwart von Säuren bei Temperaturen zwischen 0 und 100 °C, in Abwesenheit von Säuren bei Temperaturen zwischen 100 und 250 °C, gegebenenfalls auch in Gegenwart von Basen, cyclisiert wird und eine so erhaltene Verbindung, in der $R_2$ eine Alkyl- oder Aralkylgruppe und/oder $R_{21}$ eine Alkoxy- oder Aralkoxygruppe bedeuten, anschliessend einer Etherspaltung unterworfen wird, und gewünschtenfalls anschliessend eine so erhaltene Verbindung der allgemeinen Formel I mit Alkali- oder Erdalkalibasen in die entsprechenden Salze übergeführt wird.

9. Verfahren gemäss Anspruch 8a, dadurch gekennzeichnet, dass die Verseifung mit einer wässerigen Natrium- oder Kaliumhydroxidlösung erfolgt und die Etherspaltung, falls $R_2$ eine Alkyl- oder Aralkylgruppe und/oder $R_{21}$ eine Alkoxy- oder Aralkoxygruppe bedeuten, entweder mittels Lewis-Säuren in aprotischen Lösungsmitteln bei Temperaturen bis zum Siedepunkt des Reaktionsgemisches, bei Verwendung von Pyridinhydrochlorid in der Schmelze, oder mittels Basen in polaren aprotischen Lösungsmitteln in Gegenwart von Alkali- oder Erdalkalihalogeniden oder -pseudohalogeniden bei Temperaturen zwischen 120 und 200 °C durchgeführt wird.

10. Verfahren gemäss Anspruch 8b, dadurch gekennzeichnet, dass die Oxidation einer Verbindung der allgemeinen Formel III mittels einer wässerigen, alkalischen Kaliumpermanganatlösung oder mittels einer wässerigen sauren Kaliumdichromatlösung bei Temperaturen zwischen 60 und 90 °C durchgeführt wird und die anschliessende Etherspaltung entweder mittels Lewis-Säuren in aprotischen Lösungsmitteln bei Temperaturen bis zum Siedepunkt des Reaktionsgemisches, mittels Pyridinhydrochlorid in der Schmelze, oder mittels Basen in polaren aprotischen Lösungsmitteln in Gegenwart von Alkali- oder Erdalkalihalogeniden oder -pseudohalogeniden bei Temperaturen zwischen 120 und 200 °C erfolgt.

11. Verfahren gemäss Anspruch 8c, dadurch gekennzeichnet, dass bei Vorliegen von Verbindungen der allgemeinen Formel IV, in der $R_4$ ein Halogenatom oder ein Pseudohalogen darstellt, diese nukleophil austauschbare Gruppe gegen eine Hydroxygruppe durch Erhitzen der Verbindung in einem Druckgefäss in Anwesenheit von Wasser, einem Alkalihydroxid oder -carbonat und einem Katalysator auf Temperaturen zwischen 140 und 180 °C ausgetauscht wird, wobei als Katalysator vorzugsweise Kupferpulver, ein Kupfer(I)- oder ein Kupfer(II)salz oder ein Gemisch dieser Katalysatoren verwendet wird.

12. Verfahren gemäss Anspruch 8d, dadurch gekennzeichnet, dass eine Verbindung der allgemeinen Formel V zur Cyclisierung entweder mit Phosphorsäure, Polyphosphorsäure, Schwefelsäure oder mit Gemischen dieser Säuren bei 50 bis 70 °C behandelt oder auf Temperaturen zwischen 100 und 250 °C in Gegenwart von Lösungsmitteln erhitzt oder mit Alkalialkoholaten zur Reaktion gebracht wird und die anschliessende Etherspaltung entweder mittels Lewis-Säuren in aprotischen Lösungsmitteln bei Temperaturen bis zum Siedepunkt des Reaktionsgemisches, mittels Pyridinhydrochlorid in der Schmelze, oder mittels Basen in polaren aprotischen Lösungsmitteln in Gegenwart von Alkali- oder Erdalkalihalogeniden oder -pseudohalogeniden bei Temperaturen zwischen 120 und 200 °C durchgeführt wird.

**Patentansprüche für den Vertragsstaat: AT**

1. Verfahren zur Herstellung von 4-Hydroxy-1,2-benzisothiazol-3(2H)-on-1,1-dioxiden der allgemeinen Formel I

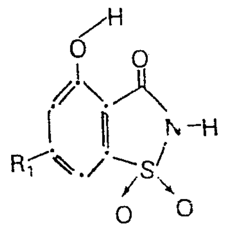

(I)

in der $R_1$ ein Wasserstoffatom oder die Hydroxygruppe bedeutet und von deren Alkali- oder Erdalkalisalzen, dadurch gekennzeichnet, dass

a) ein 3-Amino-1,2-benzisothiazol-1,1-dioxid der allgemeinen Formel II

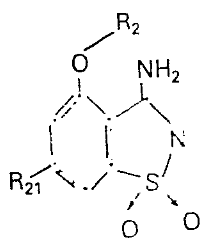

(II)

in der $R_2$ ein Wasserstoffatom, eine Alkyl- oder Aralkylgruppe und $R_{21}$ ein Wasserstoffatom, eine Hydroxy-, Alkoxy- oder Aralkoxygruppe bedeuten, in einer wässerigen Lösung mit Basen bei Temperaturen zwischen 50 °C und dem Siedepunkt der Lösung verseift wird und, falls $R_2$ eine Alkyl- oder Aralkylgruppe und/oder $R_{21}$ eine Alkoxy- oder Aralkoxygruppe bedeuten, das so erhaltene Produkt anschliessend einer Etherspaltung unterworfen wird oder

b) ein o-Sulfamoyl-o'-alkoxy(oder o'-aralkoxy)-toluol der allgemeinen Formel III

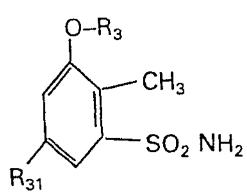

(III)

in der $R_3$ eine Alkyl- oder Aralkylgruppe und $R_{31}$ ein Wasserstoffatom oder eine Alkoxy- oder Aralkoxygruppe bedeuten, mit einem üblichen Oxidationsmittel oxidiert und das so erhaltene Produkt anschliessend einer Etherspaltung unterworfen wird, oder

c) in einem 1,2-Benzisothiazol-3(2H)-on-1,1-dioxid der allgemeinen Formel IV

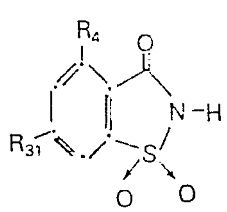

(IV)

in der $R_{31}$ wie oben definiert ist, und $R_4$ eine nucleophil austauschbare Gruppe bedeutet, der Rest $R_4$ gegen die Hydroxygruppe nucleophil ausgetauscht wird, wobei der Austausch eines Diazoniumkations in einer sauren wässerigen Lösung bei der Siedetemperatur der Lösung und der Austausch anderer austauschbarer Gruppen mit Alkalibasen in Gegenwart von Katalysatoren und in Anwesenheit von Wasser bei Temperaturen zwischen 120 und 200 °C erfolgt, und, falls $R_{31}$ eine Alkoxy- oder Aralkoxygruppe darstellt, das so erhaltene Produkt anschliessend einer Ätherspaltung unterworfen wird, oder

d) ein 2-Sulfamoyl-3-carbonsäurederivat der allgemeinen Formel V

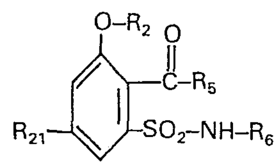

(V)

in der $R_{21}$ ein Wasserstoffatom, eine Hydroxy-, Alkoxy- oder Aralkoxygruppe und $R_2$ ein Wasserstoffatom oder eine Alkyl- oder Aralkylgruppe bedeutet, $R_6$ ein Wasserstoffatom oder einen tert.Alkylrest mit 4 bis 19 Kohlenstoffatomen und $R_5$ eine Hydroxygruppe, eine Alkoxygruppe mit 1 bis 10 Kohlenstoffatomen, eine Aryloxygruppe, worin Aryl für Phenyl oder Naphthyl steht, eine Phenyl- oder Naphthylalkoxygruppe mit 1 bis 3 Kohlenstoffatomen im Alkylenteil oder ein Halogenatom oder eine andere, als nucleophil austauschbar vorbekannte Gruppe bedeuten, in Gegenwart von Säuren bei Temperaturen zwischen 0 und 100 °C, in Abwesenheit von Säuren bei Temperaturen zwischen 100 und 250 °C, gegebenenfalls auch in Gegenwart von Basen, cyclisiert wird und eine so erhaltene Verbindung, in der $R_2$ eine Alkyl- oder Aralkylgruppe und/oder $R_{21}$ eine Alkoxy- oder Aralkoxygruppe bedeuten, anschliessend einer Etherspaltung unterworfen wird, und gewünschtenfalls anschliessend eine so erhaltene Verbindung der allgemeinen Formel I mit Alkali- oder Erdalkalibasen in die entsprechenden Salze übergeführt wird.

2. Verfahren gemäss Anspruch 1a, dadurch gekennzeichnet, dass die Verseifung mit einer wässerigen Natrium- oder Kaliumhydroxidlösung erfolgt und die Etherspaltung, falls $R_2$ eine Alkyl- oder Aralkylgruppe und/oder $R_{21}$ eine Alkoxy- oder Aralkoxygruppe bedeuten, entweder mittels Lewis-Säuren in aprotischen Lösungsmitteln bei Temperaturen bis zum Siedepunkt des Reaktionsgemisches, bei Verwendung von Pyridinhydrochlorid in der Schmelze, oder mittels Basen in polaren aprotischen Lösungsmitteln in Gegenwart von Alkali- oder Erdalkalihalogeniden oder -pseudohalogeniden bei Temperaturen zwischen 120 und 200 °C durchgeführt wird.

3. Verfahren gemäss Anspruch 1b, dadurch gekennzeichnet, dass die Oxidation einer Verbindung der allgemeinen Formel III mittels einer wässerigen, alkalischen Kaliumpermanganatlösung

oder mittels einer wässerigen sauren Kaliumdichromatlösung bei Temperaturen zwischen 60 und 90 °C durchgeführt wird und die anschliessende Etherspaltung entweder mittels Lewis-Säuren in aprotischen Lösungsmitteln bei Temperaturen bis zum Siedepunkt des Reaktionsgemisches, mittels Pyridinhydrochlorid in der Schmelze, oder mittels Basen in polaren aprotischen Lösungsmitteln in Gegenwart von Alkali- oder Erdalkalihalogeniden oder -pseudohalogeniden bei Temperaturen zwischen 120 und 200 °C erfolgt.

4. Verfahren gemäss Anspruch 1c, dadurch gekennzeichnet, dass bei Vorliegen von Verbindungen der allgemeinen Formel IV, in der $R_4$ ein Halogenatom oder ein Pseudohalogen darstellt, diese nukleophil austauschbare Gruppe gegen eine Hydroxygruppe durch Erhitzen der Verbindung in einem Druckgefäss in Anwesenheit von Wasser, einem Alkalihydroxid oder -carbonat und einem Katalysator auf Temperaturen zwischen 140 und 180 °C ausgetauscht wird, wobei als Katalysator vorzugsweise Kupferpulver, ein Kupfer(I)- oder ein Kupfer(II)salz oder ein Gemisch dieser Katalysatoren verwendet wird.

5. Verfahren gemäss Anspruch 1d, dadurch gekennzeichnet, dass eine Verbindung der allgemeinen Formel V zur Cyclisierung entweder mit Phosphorsäure, Polyphosphorsäure, Schwefelsäure oder mit Gemischen dieser Säuren bei 50 bis 70 °C behandelt oder auf Temperaturen zwischen 100 und 250 °C in Gegenwart von Lösungsmitteln erhitzt oder mit Alkalialkoholaten zur Reaktion gebracht wird und die anschliessende Etherspaltung entweder mittels Lewis-Säuren in aprotischen Lösungsmitteln bei Temperaturen bis zum Siedepunkt des Reaktionsgemisches, mittels Pyridinhydrochlorid in der Schmelze, oder mittels Basen in polaren aprotischen Lösungsmitteln in Gegenwart von Alkali- oder Erdalkalihalogeniden oder -pseudohalogeniden bei Temperaturen zwischen 120 und 200 °C durchgeführt wird.

**Revendications pour les Etats contractants: BE, CH, DE, FR, GB, IT, LI, LU, NL, SE.**

1. 1,1-dioxydes de 4-hydroxy-1,2-benzisothiazol-3(2H)-ones de formule générale I

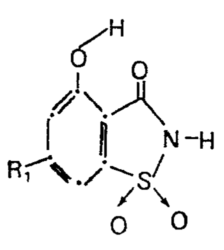

(I)

dans laquelle $R_1$ représente un atome d'hydrogène ou le groupe hydroxy, et leurs sels alcalins ou alcalino-terreux physiologiquement inoffensifs.

2. Le 1,1-dioxyde de 4-hydroxy-1,2-benzisothiazol-3(2H)-one et ses sels alcalins ou alcalino-terreux physiologiquement inoffensifs.

3. Le 1,1-dioxyde de 4,6-dihydroxy-1,2-benzisothiazol-3(2H)-one et ses sels alcalins ou alcalino-terreux physiologiquement inoffensifs.

4. Préparations à base de matière édulcorante contenant un ou plusieurs composés de formule générale I selon la revendication 1, conjointement aux excipients et/ou adjuvants habituels.

5. Préparations à base de matière édulcorante contenant le sel de sodium ou le sel de calcium du 1,1-dioxyde de 4-hydroxy-1,2-benzisothiazol-3(2H)-one.

6. Préparations à base de matière édulcorante contenant le sel de sodium ou le sel de calcium du 1,1-dioxyde de 4,6-dihydroxy-1,2-benzisothiazol-3(2H)-one.

7. Utilisation des composés de formule générale I et/ou de leurs sels physiologiquement supportables selon la revendication 1, pour l'obtention de préparations à base de matière édulcorante.

8. Procédé pour la préparation de 1,1-dioxydes de 4-hydroxy-1,2-benzisothiazol-3(2H)-ones de formule générale I selon la revendication 1, caractérisé en ce que

a) on saponifie un 1,1-dioxyde de 3-amino-1,2-benzisothiazole de formule générale II

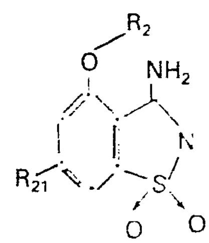

(II)

dans laquelle $R_2$ représente un atome d'hydrogène, un groupe alcoyle ou aralcoyle et $R_{21}$ représente un atome d'hydrogène, un groupe hydroxy, alcoxy ou aralcoxy, dans une solution aqueuse avec des bases à des températures entre 50 °C et le point d'ébullition de la solution et, dans le cas où $R_2$ représente un groupe alcoyle ou aralcoyle et/ou $R_{21}$ représente un groupe alcoxy ou aralcoxy, on soumet ensuite le produit ainsi obtenu à un clivage d'éther, ou

b) on oxyde un o-sulfamoyl-o'-alcoxy (ou o'-aralcoxy)-toluène de formule générale III

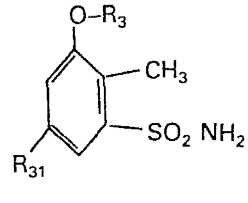

(III)

dans laquelle $R_3$ représente un groupe alcoyle ou aralcoyle et $R_{31}$ représente un atome d'hydrogène ou un groupe alcoxy ou aralcoxy, au moyen d'un agent d'oxydation habituel et on soumet ensuite le produit ainsi obtenu à un clivage d'éther, ou

c) dans un 1,1-dioxyde de 1,2-benzisothiazol-3(2H)-one de formule générale IV

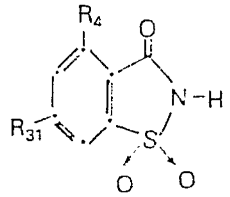

(IV)

dans laquelle $R_{31}$ est défini comme plus haut et $R_4$ représente un groupe échangeable de façon nucléophile, on échange de façon nucléophile le radical $R_4$ contre le groupe hydroxy, l'échange d'un cation diazonium ayant lieu dans une solution acide aqueuse à la température d'ébullition de la solution et l'échange d'autres groupes échangeables avec des bases alcalines ayant lieu en présence de catalyseurs et en présence d'eau à des températures entre 120 et 200 °C, et, dans le cas où $R_{31}$ représente un groupe alcoxy ou aralcoxy, on soumet ensuite le produit ainsi obtenu à un clivage d'éther, ou

d) on cyclise un dérivé d'acide 2-sulfamoyl-3-carboxylique de formule générale V

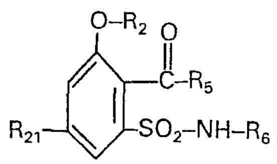

(V)

dans laquelle $R_{21}$ représente un atome d'hydrogène, un groupe hydroxy, alcoxy ou aralcoxy et $R_2$ représente un atome d'hydrogène ou un groupe alcoyle ou aralcoyle, $R_6$ représente un atome d'hydrogène ou un radical tert-alcoyle avec 4 à 19 atomes de carbone et $R_5$ représente un groupe hydroxy, un groupe alcoxy avec 1 à 10 atomes de carbone, un groupe aryloxy dans lequel aryle représente phényle ou naphtyle, un groupe phényl- ou naphtylalcoxy avec 1 à 3 atomes de carbone dans la partie alcoylène ou 1 atome d'halogène ou un autre groupe déjà connu comme échangeable de façon nucléophile, en présence d'acides à des températures entre 0 et 100 °C, en l'absence de acides à des températures entre 100 et 250 °C, éventuellement aussi en présence de bases et on soumet ensuite un composé ainsi obtenu dans lequel $R_2$ représente un groupe alcoyle ou aralcoyle et/ou $R_{21}$ représente un groupe alcoxy ou aralcoxy à un clivage d'éther, et si on le désire on transforme ensuite un composé ainsi obtenu de formule générale I au moyen de bases alcalines ou alcalino-terreuses en les sels correspondants.

9. Procédé selon la revendication 8 a), caractérisé en ce que la saponification est effectuée au moyen d'une solution aqueuse d'hydroxyde de sodium ou de potassium et en ce que le clivage d'éther, dans le cas où $R_2$ représente un groupe alcoyle ou aralcoyle et/ou $R_{21}$ représente un groupe alcoxy ou aralcoxy, est effectué soit au moyen d'acides de Lewis dans des solvants aprotiques à des températures allant jusqu'à la température d'ébullition du mélange réactionnel, en utilisant du chlorhydrate de pyridine à l'état fondu, soit au moyen de bases dans des solvants aprotiques polaires en présence d'halogénures ou de pseudohalogénures alcalins ou alcalino-terreux à des températures entre 120 et 200 °C.

10. Procédé selon la revendication 8 b), caractérisé en ce que l'oxydation d'un composé de formule générale III est effectuée au moyen d'une solution aqueuse, alcaline de permenganate de potassium ou au moyen d'une solution aqueuse, acide de bichromate de potassium à des températures entre 60 et 90 °C et en ce que le clivage d'éther subséquent est effectué soit au moyen d'acides de Lewis dans des solvants aprotiques à des températures allant jusqu'au point d'ébullition du mélange réactionnel, au moyen de chlorhydrate de pyridine à l'état fondu, soit au moyen de bases dans des solvants aprotiques polaires en présence d'halogénures ou de pseudohalogénures alcalins ou alcalino-terreux à des températures entre 120 et 200 °C.

11. Procédé selon la revendication 8 c), caractérisé en ce que lorsque l'on est en présence de composés de formule générale IV dans laquelle $R_4$ représente un atome d'halogène ou un pseudohalogène, ce groupe échangeable de façon nucléophile contre un groupe hydroxy est échangé par chauffage du composé dans un récipient sous pression en présence d'eau, d'un hydroxyde alcalin ou d'un carbonate alcalin et d'un catalyseur à des températures entre 140 et 180 °C, en utilisant comme catalyseur de préférence la poudre de cuivre, un sel de cuivre(I) ou de cuivre(II) ou un mélange de ces catalyseurs.

12. Procédé selon la revendication 8 d), caractérisé en ce que, pour la cyclisation, un composé de formule générale V est soit traité à 50 à 70 °C par l'acide phosphorique, l'acide polyphosphorique, l'acide sulfurique ou des mélanges de ces acides, soit chauffé à des températures entre 100 et 250 °C en présence de solvants ou amené à réagir avec des alcoolates alcalins et en ce que le clivage d'éther subséquent est effectué soit au moyen d'acides de Lewis dans des solvants aprotiques à des températures allant jusqu'à la température d'ébullition du mélange réactionnel, au moyen de chlorhydrate de pyridine à l'état fondu, soit au moyen de bases, dans des solvants aprotiques polaires en présence d'halogénures ou de pseudohalogénures alcalins ou alcalino-terreux à des températures entre 120 et 200 °C.

**Revendications pour l'état contractant: AT.**

1. Procédé pour la préparation de 1,1-dioxydes de 4-hydroxy-1,2-benzisothiazol-3(2H)-ones de formule générale I

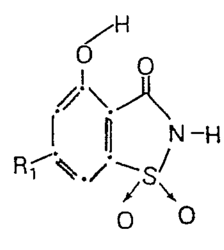

(I)

dans laquelle $R_1$ représente un atome d'hydrogène ou le groupe hydroxy et de leurs sels alcalins ou alcalino-terreux, caractérisé en ce que

a) on saponifie un 1,1-dioxyde de 3-amino-1,2-benzisothiazole de formule générale II

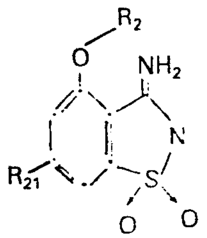

(II)

dans laquelle $R_2$ représente un atome d'hydrogène, un groupe alcoyle ou aralcoyle et $R_{21}$ représente un atome d'hydrogène, un groupe hydroxy, alcoxy ou aralcoxy, dans une solution aqueuse avec des bases à des températures entre 50 °C et le point d'ébullition de la solution et, dans le cas où $R_2$ représente un groupe alcoyle ou aralcoyle et/ou $R_{21}$ représente un groupe alcoxy ou aralcoxy, on soumet ensuite le produit ainsi obtenu à un clivage d'éther, ou

b) on oxyde un o-sulfamoyl-o'-alcoxy (ou o'-aralcoxy)-toluène de formule générale III

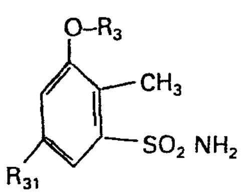

(III)

dans laquelle $R_3$ représente un groupe alcoyle ou aralcoyle et $R_{31}$ représente un atome d'hydrogène ou un groupe alcoxy ou aralcoxy, au moyen d'un agent d'oxydation habituel et on soumet ensuite le produit ainsi obtenu à un clivage d'éther, ou

c) dans un 1,1-dioxyde de 1,2-benzisothiazol-3(2H)-one de formule générale IV

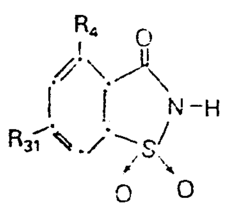

(IV)

dans laquelle $R_{31}$ est défini comme plus haut et $R_4$ représente un groupe échangeable de façon nucléophile, on échange de façon nucléophile le radical $R_4$ contre le groupe hydroxy, l'échange d'un cation diazonium ayant lieu dans une solution acide aqueuse à la température d'ébullition de la solution et l'échange d'autres groupes échangeables ayant lieu au moyen de bases alcalines en présence de catalyseurs et en présence d'eau à des températures entre 120 et 200 °C, et, dans le cas où $R_{31}$ représente un groupe alcoxy ou aralcoxy, on soumet ensuite le produit ainsi obtenu à un clivage d'éther, ou

d) on cyclise un dérivé d'acide 2-sulfamoyl-3-carboxylique de formule générale V

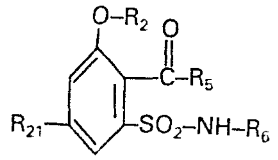

(V)

dans laquelle $R_{21}$ représente un atome d'hydrogène, un groupe hydroxy, alcoxy ou aralcoxy et $R_2$ représente un atome d'hydrogène ou un groupe alcoyle ou aralcoyle, $R_6$ représente un atome d'hydrogène ou un radical tert-alcoyle avec 4 à 19 atomes de carbone et $R_5$ représente un groupe hydroxy, un groupe alcoxy avec 1 à 10 atomes de carbone, un groupe aryloxy dans lequel aryle représente phényle ou naphtyle, un groupe phényl- ou naphtylalcoxy avec 1 à 3 atomes de carbone dans la partie alcoylène ou un atome d'halogène ou un autre groupe, déjà connu, échangeable de façon nucléophile, en présence d'acides à des températures entre 0 et 100 °C, en l'absence de acides à des températures entre 100 et 250 °C, éventuellement aussi en présence de bases et ce qu'on soumet ensuite un composé ainsi obtenu dans lequel $R_2$ représente un groupe alcoyle ou aralcoyle et/ou $R_{21}$ représente un groupe alcoxy ou aralcoxy à un clivage d'éther, et ce qu'éventuellement on transforme ensuite un composé ainsi obtenu de formule générale I au moyen de bases alcalines ou alcalino-terreuses en les sels correspondants.

2. Procédé selon la revendication 1 a), caractérisé en ce que la saponification est effectuée au moyen d'une solution d'hydroxyde de sodium ou d'hydroxyde de potassium et en ce que le clivage d'éther, dans le cas où $R_2$ représente un groupe alcoyle ou aralcoyle et/ou $R_{21}$ représente un groupe alcoxy ou aralcoxy, est effectué soit au moyen d'acides de Lewis dans des solvants aprotiques à des températures allant jusqu'au point d'ébullition du mélange réactionnel, en utilisant du chlorhydrate de pyridine à l'état fondu, soit au moyen de bases dans des solvants aprotiques polaires en présence d'halogénures ou de pseudohalogénures alcalins ou alcalino-terreux à des températures entre 120 et 200 °C.

3. Procédé selon la revendication 1 b), caractérisé en ce que l'oxydation d'un composé de formule générale III est effectuée au moyen d'une solution aqueuse, alcaline de permenganate de potassium ou au moyen d'une solution aqueuse, acide de bichromate de potassium à des températures entre 60 et 90 °C et en ce que le clivage d'éther subséquent a lieu soit au moyen d'acides de Lewis dans des solvants aprotiques à des températures allant jusqu'au point d'ébullition du mélange réactionnel, au moyen de chlorhydrate de pyridine à l'état fondu, soit au moyen de bases dans des solvants polaires aprotiques en présence d'halogénures ou de pseudohalogénures alcalins ou alcalinoterreux à des températures entre 120 et 200 °C.

4. Procédé selon la revendication 1 c), caractérisé en ce que lorsqu'on est en présence de composés de formule générale IV dans laquelle $R_4$ représente un atome d'halogène ou un pseudohalogène, ce groupe échangeable de façon nucléophile contre un groupe hydroxy est échangé à des températures entre 140 et 180 °C par chauffage du composé dans un récipient sous pression en présence d'eau, d'un hydroxyde alcalin ou d'un carbonate alcalin et d'un catalyseur, en utilisant comme catalyseur de préférence de la poudre de cuivre, un sel de cuivre(I) ou de cuivre(II) ou un mélange de ces catalyseurs.

5. Procédé selon la revendication 1 d), caractérisé en ce que, pour la cyclisation, un composé de formule générale V est soit traité à 50 à 70 °C par l'acide phosphorique, l'acide polyphosphorique, l'acide sulfurique ou des mélanges de ces acides, soit chauffé à des températures entre 100 et 250 °C en présence de solvants ou amené à réagir avec des alcoolates alcalins et en ce que le clivage d'éther subséquent est effectué soit au moyen d'acides de Lewis dans des solvants aprotiques à des températures allant jusqu'au point d'ébullition du mélange réactionnel, au moyen de chlorhydrate de pyridine à l'état fondu, soit au moyen de bases dans des solvants aprotiques polaires en présence d'halogénures ou de pseudohalogénures alcalins ou alcalino-terreux à des températures entre 120 et 200 °C.

**Claims for the contracting states: BE, CH, DE, FR, GB, IT, LI, LU, NL, SE**

1. 4-Hydroxy-1,2-benzisothiazol-3(2H)-one-1,1-dioxides of general formula I

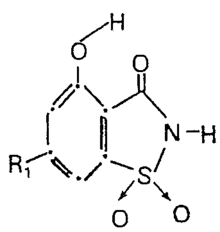

(I)

wherein $R_1$ represents a hydrogen atom or a hydroxy group, and the physiologically acceptable alkali or alkaline earth metal salts thereof.

2. 4-Hydroxy-1,2-benzisothiazol-3(2H)-one-1,1-dioxide and the physiologically acceptable alkali or alkaline earth metal salts thereof.

3. 4,6-Dihydroxy-1,2-benzisothiazol-3(2H)-one-1,1-dioxide and the physiologically acceptable alkali or alkaline earth metal salts thereof.

4. Sweetening preparations, containing one or more compounds of general formula I as claimed in claim 1 together with the conventional carriers and/or excipients.

5. Sweetening preparations containing the sodium or calcium salt of 4-hydroxy-1,2-benzisothiazol-3(2H)-one-1,1-dioxide.

6. Sweetening preparations containing the sodium or calcium salt of 4,6-dihydroxy-1,2-benzisothiazol-3(2H)-one-1,1-dioxide.

7. Use of the compounds of general formula I and/or of the physiologically acceptable salts thereof as claimed in claim 1 for the preparation of sweetening compositions.

8. Process for preparing 4-Hydroxy-1,2-benzisothiazol-3(2H)-one-1,1-dioxides of general formula I as claimed in claim 1, characterised in that

a) a 3-amino-1,2-benzisothiazole-1,1-dioxide of general formula II

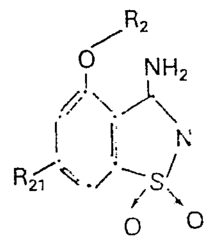

(II)

wherein $R_2$ represents a hydrogen atom, an alkyl or aralkyl group and $R_{21}$ represents a hydrogen atom, a hydroxy, alkoxy or aralkoxy group, is saponified in an aqueous solution with bases, at temperatures of between 50 °C and the boiling point of the solution, and if $R_2$ represents an alkyl or aralkyl group and/or $R_{21}$ represents an alkoxy or aralkoxy group, the product thus obtained is subsequently subjected to ether splitting, or

b) an o-sulfamoyl-o'-alkoxy (or o'-aralkoxy)-toluene of general formula

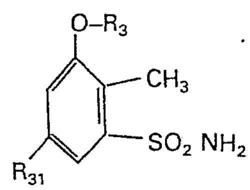

(III)

wherein $R_3$ represents an alkyl or aralkyl group and $R_{31}$ represents a hydrogen atom or an alkoxy or aralkoxy group, is oxidised with a conventional oxidising agent and the product thus obtained is subsequently subjected to ether splitting, or

c) in a 1,2-benzisothiazol-3(2H)-one-1,1-dioxide of general formula

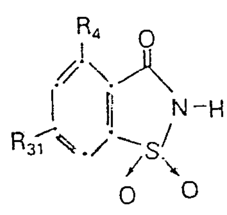

(IV)

wherein $R_{31}$ is as hereinbefore defined and $R_4$ represents a nucleophilically exchangeable group, the group $R_4$ is nucleophilically exchanged for the hydroxy group, the exchange of a diazonium cation being carried out in an aqueous acid solution at the boiling temperature of the solution whilst the exchange of other exchangeable groups is effected with alkali metal bases in the presence of catalysts and in the presence of water at temperatures of between 120 and 200 °C and, if $R_{31}$ repre-

sents an alkoxy or aralkoxy group, the product thus obtained is subsequently subjected to ether splitting, or

d) a 2-sulfamoyl-3-carboxylic acid derivative of general formula V

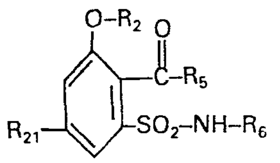

(V)

wherein $R_{21}$ represents a hydrogen atom, a hydroxy, alkoxy or aralkoxy group and $R_2$ represents a hydrogen atom or an alkyl or aralkyl group, $R_6$ represents a hydrogen atom or a tert.alkyl group with 4 to 19 carbon atoms and $R_5$ represents a hydroxy group, an alkoxy group with 1 to 10 carbon atoms, an aryloxy group, wherein aryl represents phenyl or naphthyl, a phenylalkoxy or naphthylalkoxy group with 1 to 3 carbon atoms in the alkylene moiety or a halogen atom or another group known to be nucleophilically exchangeable, is cyclised in the presence of acids at temperatures of between 0 and 100 °C, in the absence of acids at temperatures of between 100 and 250 °C, and possibly also in the presence of bases, and a compound thus obtained wherein $R_2$ represents an alkyl or aralkyl group and/or $R_{21}$ represents an alkoxy or aralkoxy group is subsequently subjected to ether splitting, and if desired a compound of general formula I thus obtained is subsequently converted into the corresponding salts thereof with alkali or alkaline earth metal bases.

9. Process as claimed in claim 8a, characterised in that the saponification is effected with an aqueous sodium or potassium hydroxide solution and, if $R_2$ represents an alkyl or aralkyl group and/or $R_{21}$ represents an alkoxy or aralkoxy group, the ether splitting is effected either using Lewis acids in aprotic solvents at temperatures up to the boiling point of the reaction mixture, or using pyridine hydrochloride in a melt, or by means of bases in polar aprotic solvents in the presence of alkali or alkaline earth metal halides or pseudohalides at temperatures of between 120 and 200 °C.

10. Process as claimed in claim 8b, characterised in that the oxidation of a compound of general formula III is effected by means of an aqueous alkaline potassium permanganate solution or using an aqueous acidic potassium dichromate solution at temperatures of between 60 and 90 °C and the subsequent ether splitting is effected either using Lewis acids in aprotic solvents at temperatures up to the boiling point of the reaction mixture, using pyridine hydrochloride in a melt, or using bases in polar aprotic solvents in the presence of alkali or alkaline earth metal halides or pseudohalides at temperatures of between 120 and 200 °C.

11. Process as claimed in claim 8c, characterised in that if compounds of general formula IV are present wherein $R_4$ represents a halogen atom or a pseudohalogen, this nucleophilically exchangeable group is exchanged for a hydroxy group by heating the compound in a pressure vessel in the presence of water, an alkali metal hydroxide or carbonate and a catalyst to temperatures of between 140 and 180 °C, whilst the catalyst used is preferably copper powder, a copper(I) or copper (II) salt or a mixture of these catalysts.

12. Process as claimed in claim 8d, characterised in that a compound of general formula V is cyclised either by treating it with phosphoric, polyphosphoric or sulphuric acid or with mixtures of these acids at 50 to 70 °C or by heating it to temperatures of between 100 and 250 °C in the presence of solvents or by reacting it with alkali metal alkoxides, and the subsequent ether splitting is effected either by means of Lewis acids in aprotic solvents at temperatures up to the boiling point of the reaction mixture, using pyridine hydrochloride in a melt, or using bases in polar aprotic solvents in the presence of alkali or alkaline earth metal halides or pseudohalides at temperatures of between 120 and 200 °C.

**Claims for the contracting state: AT**

1. Process for preparing 4-hydroxy-1,2-benzisothiazol-3(2H)-one-1,1-dioxides of general formula I

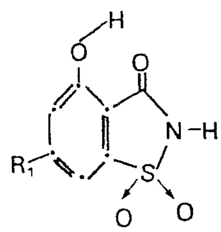

(I)

wherein $R_1$ represents a hydrogen atom or a hydroxy group and of the alkali or alkaline earth metal salts thereof, characterised in that

a) a 3-amino-1,2-benzisothiazole-1,1-dioxide of general formule II

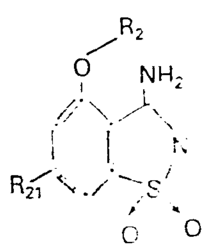

(II)

wherein $R_2$ represents a hydrogen atom, an alkyl or aralkyl group and $R_{21}$ represents a hydrogen atom, a hydroxy, alkoxy or aralkoxy group, is saponified in an aqueous solution with bases, at temperatures of between 50 °C and the boiling point of the solution, and if $R_2$ represents an alkyl or aralkyl group and/or $R_{21}$ represents an alkoxy or aralkoxy group, the product thus obtained is subsequently subjected to ether splitting, or

b) an o-sulfamoyl-o'-alkoxy (or o'-aralkoxy)-toluene of general formula

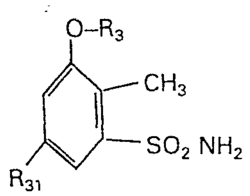

(III)

wherein $R_3$ represents an alkyl or aralkyl group and $R_{31}$ represents a hydrogen atom or an alkoxy or aralkoxy group, is oxidised with a conventional oxidising agent and the product thus obtained is subsequently subjected to ether splitting, or

c) in a 1,2-benzisothiazol-3(2H)-one-1,1-dioxide of general formula

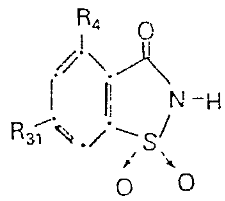

(IV)

wherein $R_{31}$ is as hereinbefore defined and $R_4$ represents a nucleophilically exchangeable group, the group $R_4$ is nucleophilically exchanged for the hydroxy group, the exchange of a diazonium cation being carried out in an aqueous acid solution at the boiling temperature of the solution whilst the exchange of other exchangeable groups is effected with alkali metal bases in the presence of catalysts and in the presence of water at temperatures of between 120 and 200 °C and, if $R_{31}$ represents an alkoxy or aralkoxy group, the product thus obtained is subsequently subjected to ether splitting, or

d) a 2-sulfamoyl-3-carboxylic acid derivative of general formula V

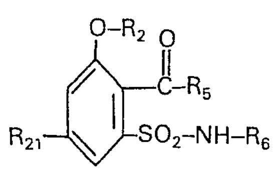

(V)

wherein $R_{21}$ represents a hydrogen atom, a hydroxy, alkoxy or aralkoxy group and $R_2$ represents a hydrogen atom or an alkyl or aralkyl group, $R_6$ represents a hydrogen atom or a tert.alkyl group with 4 to 19 carbon atoms and $R_5$ represents a hydroxy group, an alkoxy group with 1 to 10 carbon atoms, an aryloxy group, wherein aryl represents phenyl or naphthyl, a phenylalkoxy or naphthylalkoxy group with 1 to 3 carbon atoms in the alkylene moiety or a halogen atom or another group known to be nucleophilically exchangeable, is cyclised in the presence of acids at temperatures of between 0 and 100 °C, in the absence of acids at temperatures of between 100 and 250 °C, and possibly also in the presence of bases, and a compound thus obtained wherein $R_2$ represents an alkyl or aralkyl group and/or $R_{21}$ represents an alkoxy or aralkoxy group is subsequently subjected to ether splitting, and if desired a compound of general formula I thus obtained is subsequently converted into the corresponding salts thereof with alkali or alkaline earth metal bases.

2. Process as claimed in claim 1a, characterised in that the saponification is effected with an aqueous sodium or potassium hydroxide solution and, if $R_2$ represents an alkyl or aralkyl group and/or $R_{21}$ represents an alkoxy or aralkoxy group, the ether splitting is effected either using Lewis acids in aprotic solvents at temperatures up to the boiling point of the reaction mixture, using pyridine hydrochloride in a melt, or by means of bases in polar aprotic solvents in the presence of alkali or alkaline earth metal halides or pseudohalides at temperatures of between 120 and 200 °C.

3. Process as claimed in claim 1b, characterised in that the oxidation of a compound of general formula III is effected by means of an aqueous alkaline potassium permanganate solution or using an aqueous acidic potassium dichromate solution at temperatures of between 60 and 90 °C and the subsequent ether splitting is effected either using Lewis acids in aprotic solvents at temperatures up to the boiling point of the reaction mixture, using pyridine hydrochloride in a melt, or using bases in polar aprotic solvents in the presence of alkali or alkaline earth metal halides or pseudohalides at temperatures of between 120 and 200 °C.

4. Process as claimed in claim 1c, characterised in that if compounds of general formula IV are present wherein $R_4$ represents a halogen atom or a pseudohalogen, this nucleophilically exchangeable group is exchanged for a hydroxy group by heating the compound in a pressure vessel in the presence of water, an alkali metal hydroxide or carbonate and a catalyst to temperatures of between 140 and 180 °C, whilst the catalyst used is preferably copper powder, a copper(I) or copper(II) salt or a mixture of these catalysts.

5. Process as claimed in claim 1d, characterised in that a compound of general formula V is cyclised either by treating it with phosphoric, polyphosphoric or sulphuric acid or with mixtures of these acids at 50 to 70 °C or by heating it to temperatures of between 100 and 250 °C in the presence of solvents or by reacting it with alkali metal alkoxides, and the subsequent ether splitting is effected either by means of Lewis acids in aprotic solvents at temperatures up to the boiling point of the reaction mixture, using pyridine hydrochloride in a melt, or using bases in polar aprotic solvents in the presence of alkali or alkaline earth metal halides or pseudohalides at temperatures of between 120 and 200 °C.